# EUROPÄISCHE PATENTSCHRIFT

(11) **EP 1 756 049 B1**
(45) Veröffentlichungstag und Bekanntmachung des Hinweises auf die Patenterteilung: **31.10.2007**
(21) Anmeldenummer: 05747892.7
(22) Anmeldetag: 20.05.2005
(51) Int. Cl.: C07D 205/08

(54) **VERFAHREN ZUR HERSTELLUNG VON 1,4-DIPHENYLAZETIDINON-DERIVATEN**
METHOD FOR PRODUCING 1,4-DIPHENYL AZETIDINONE DERIVATIVES
PROCEDE POUR PRODUIRE DES DERIVES DE 1,4-DIPHENYLAZETIDINONE

(30) Priorität: 21.05.2004 DE 102004025071; 09.03.2005 DE 102005010770
(43) Veröffentlichungstag der Anmeldung: 28.02.2007
(73) Patentinhaber: Sanofi-Aventis Deutschland GmbH, 65929 Frankfurt am Main (DE)
(72) Erfinder: LINDENSCHMIDT, Andreas, 65812 Bad Soden (DE); WILL, David, William, 65926 Frankfurt (DE); JAEHNE, Gerhard, 65929 Frankfurt (DE); WOLLMANN, Theodor, Andreas, 65796 Hattersheim (DE); FRICK, Wendelin, 65510 Hünstetten-Beuerbach (DE); JUNKER, Bernd, 65812 Bad Soden (DE); RIGAL, David, 65926 Frankfurt (DE); BILLEN, Guenter, 65527 Niedernhausen (DE); JENDRALLA, Heiner, 65931 Frankfurt (DE)
(86) Internationale Anmeldenummer: PCT/EP2005/005498
(87) Internationale Veröffentlichungsnummer: WO 2005/113496

(56) Entgegenhaltungen:
- EP-A- 1 362 855
- WO-A-02/50027
- WO-A-93/02048
- WO-A-95/01961
- WO-A-95/08532
- US-A- 5 631 365

## Beschreibung

Die Erfindung betrifft die Herstellung von 1,4-Diphenylazetidiaon-Derivaten durch Zyklisierung von β-substituierten Aminoamiden in Gegenwart von Silylierungsmitteln und Zyklisierungskatalysatoren.

Ezetimibe als bekannter Vertreter dieser Verbindungen blockiert die Resorption von Cholesterin aus dem Darm, so dass bei Patienten sowohl niedrigere LDL-Werte als auch weniger Triglyceride beobachtet werden. Dabei handelt es sich um das 1-(4-fluorphenyl)-3(R)-[3-(4-fluorphenyl)-3(S)-hydroxypropyl]-4(S)-(4-hydroxyphenyl)-2-azetidinon der nachfolgenden Formel (siehe Anspruch 8 in EP 0 720 599 B1).

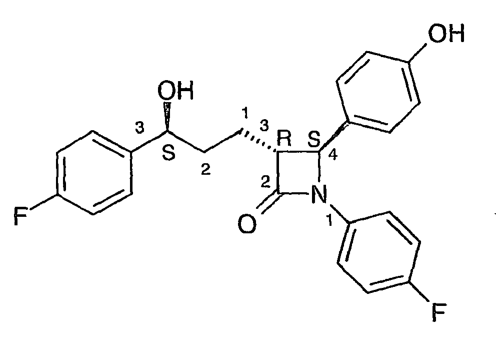

Zu dieser Verbindung selbst, einigen chemischen Abwandlungen, ihrer Herstellung nach verschiedenen Verfahrensvarianten und ihrem therapeutischen Einsatz zur Behandlung von Hyperlipidämie sowie Arteriosklerose und Hypercholesterinämie sind u. a. die folgenden Druckschriften erschienen, wobei beispielsweise versucht worden ist, chemische Abwandlungen vergleichbarer therapeutischer Wirkung, aber mit geringerer intestinaler Resorption aufzufinden.

In der EP 0 524 595 A1 werden chemische Abwandlungen zu Ezetimibe der allgemeinen Formel 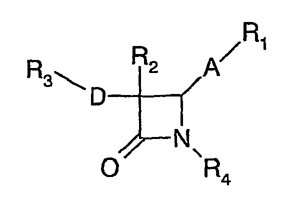 beschrieben, die u.a. in 3-Stellung des Azetidinon-Rings noch einen zweiten Substituenten (R₂), zwischen dem Phenylring in 4-Stellung des Azetidinon-Rings und dem Ring noch verbindende Molekülteile (A) und statt der Fluorgruppen an den Phenylringen (R₃, R₄) keine oder andere Substituenten tragen können. Die Synthese der Verbindungen verläuft (z. B. bei R₂ = H)
durch Zyklisierung von Hydroxyamiden der allgemeinen Formeln 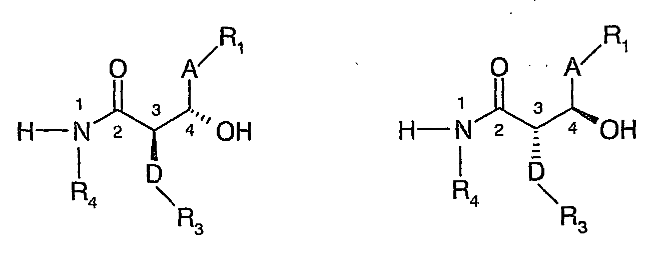 mit z.B. Trialkylphosphin/Dialkylazodicarboxylat,
Dialkylchlorphosphat/Phasentransferkatalysator, Di- oder Trichlorbenzoylchlorid/ Tetra-n-butylammoniumhydrogensulfat, oder Dichlorbenzoylchlorid/NaH.

Diese Synthesewege sind jedoch für ein großtechnisches Verfahren nicht geeignet, da z.B. Dialkylazodicarboxylate explosiv und Dialkylchlorphosphate extrem giftig sind. Bei den Synthesen mittels Di- oder Trichlorbenzoylchloriden muß entweder Tetra-n-butylammoniumhydrogensulfat in stoichometrischen Mengen oder das in großen Mengen schlecht handhabbare NaH eingesetzt werden, was beides für ein großtechnisches Verfahren ungeeignet ist.

Eine weitere in EP 0 524 595 A1 beschriebene Synthese verläuft über die Umsetzung von Carbonsäuren der nachstehenden allgemeinen Formel 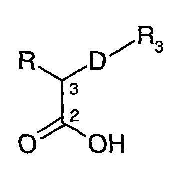 oder durch eine vergleichbare Umsetzung, aber mit einem Carbonsäurederivat, z.B. einem Säurechlorid oder gemischtem -anhydrid, mit chiralen Oxazolidinonen, zu Verbindungen der allgemeinen Formel 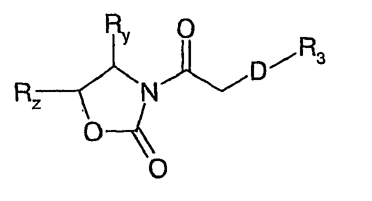 wobei Ry, R_{z} z.B. unabhängig voneinander H, C₁-C₆-Alkyl, Phenyl, Benzyl sind.

In Abwandlung der Herstellung der obigen Hydroxyamide werden Verbindungen der vorstehenden allgemeinen Formel mit Iminen in Gegenwart von TiCl₄ und TMEDA (Tetramethylethylendiamin) 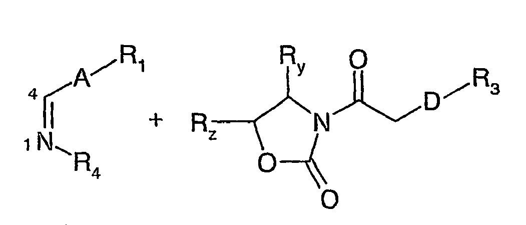 zu Verbindungen der nachstehenden allgemeinen Formel kondensiert. 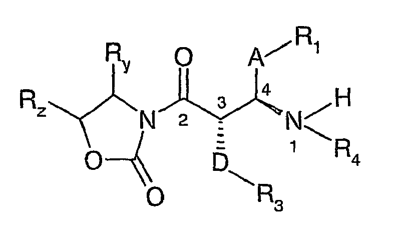 und der weiteren Umsetzung mit Na- oder Li-bistrimethylsilylamid zum 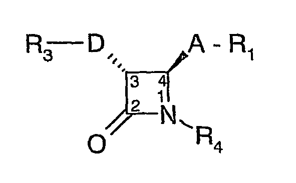 zyklisiert.

TMEDA jedoch kann bei wiederholtem Kontakt Dermatitis auslösen und ist stark wassergefährdend. Des Weiteren muss die Zyklisierung mit Na- oder Li-bistrimethylsilylamid bei tiefen Temperaturen (-78 °C) durchgeführt werden, da sonst erhebliche Mengen an Nebenprodukt gebildet werden. Daher ist dieser Syntheseweg für ein großtechnisches Verfahren nicht geeignet.

Aus der EP 0 707 567 B1 ist ein spezielles Verfahren zur Herstellung solcher Azetidinon-Derivate bekannt, bei dem (Q = H oder z. B. Alkyl) 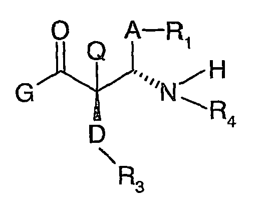 man in geeigneter Weise geschützte β-substituierte-Aminoamide der vorstehenden Formel, wobei G- u.a. einen der nachfolgenden Reste bedeutet, 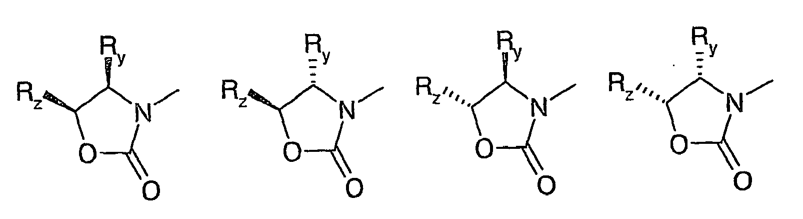 mit einem Silylierungsmittel und einem Fluoridionen-Katalysator als Zyklisierungsmittel oder einem Salz der chiralen Verbindung (G⁺-Salz) umsetzt, insbesondere mit Bis(trimethylsilyl)acetamid und Tetra-n-butylammoniumfluorid.

Ein besonderer Nachteil dieses Verfahrenswegs liegt in der Verwendung von giftigem und stark wassergefährdendem TBAF. Des Weiteren ist die hygroskopische Natur von TBAF problematisch, da ein zu hoher Wassergehalt in der Reaktionslösung zur Bildung von erheblichen Mengen eines Hydrolisierungsprodukts führt.

Weitere Verbindungsabwandlungen von Diphenylazetidinon-Derivaten sind beispielsweise in der WO 02/50027 beschrieben, wobei dort mindestens einer der Substituenten an den 3 im Molekül vorhandenen Phenylresten ein (C₁-C₃₀)-Alkylen-(LAG)-Rest ist, in dem ein oder mehrere C-Atome des Alkylenrests durch z.B. -O-, -CH=CH- oder -NR- (R=H, C₁-C₆-Alkyl, C₁-C₆-Alkylphenyl) ersetzt sein können und LAG z.B. einen Zucker-, Dizucker-, Trizucker, Aminosäure- oder Oligopeptid-Rest bedeutet.

In der WO 02/066464 werden weitere Abwandlungen von Verbindungen des 1,4-Diphenyl-azetidinon-Typs beschrieben, die durch Zyklisierung von Verbindungen der nachstehenden allgemeinen Formel 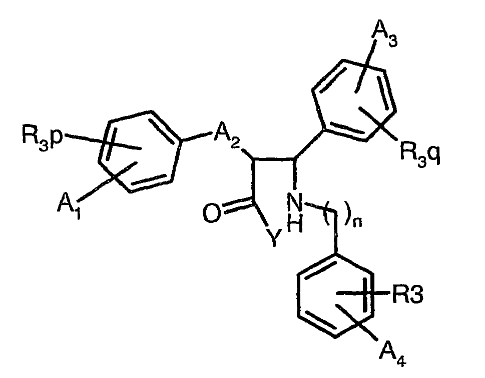 (A₁, A₃ und A₄ sind z.B. H, Halogen, C₁-C₅-Alkyl; A₂ ist z.B. eine C₁-C₅-Alkylenkette oder C₁-C₅-Alkenylenkette; R3 ist z.B. OH, OC(O)-R₁ mit R₁ z.B. H oder (C₁-C₅)Alkyl; n, p, q, r sind entweder Null oder ein Vielfaches von 1 oder 2; Y ist ein optisch aktives Sultamderivat) mit TBAF und einem Silylierungsmittel dargestellt werden.

Auf Grund der Giftigkeit und starken Wassergefährdung von TBAF ist dieses Verfahren ebenfalls nicht vorteilhaft.

Aufgabe der Erfindung ist es, eine weitere Synthesevariante für die vorstehend genannten Verbindungen aufzuzeigen, die auch stereospezifisch und in hoher Ausbeute durchgeführt werden kann, und solche Hilfsreagentien benötigt, die möglichst wenig toxisch sind. Mit Hinblick auf Verwendung in einem großtechnischen Verfahren soll auch eine Durchführung mit katalytischen Mengen an Zyklisierungsreagenz möglich sein.

Eine Lösung ist dann ein Verfahren zur Herstellung von 1,4-Diphenylazetidinon-Derivaten aus in geeigneter Weise geschützten β-substituierten Aminoamiden in Gegenwart von Silylierungsmitteln und mindestens einem Zyklisierungskatalysator, wobei dieser Zyklisierungskatalysator durch eine der nachstehenden allgemeinen Formeln 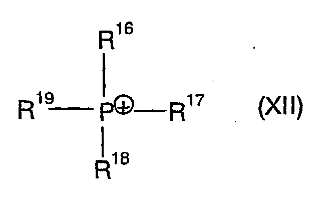 als Kation, wobei R¹⁶, R¹⁷, R¹⁸, R¹⁹ unabhängig voneinander Aryl, (C₁-C₁₅)Alkyl, Benzyl bedeuten,
R⁴¹ Aryl, (C₁-C₁₅)Alkyl, Benzyl bedeutet,
R⁴² (C₁-C₁₅)Alkyl, Benzyl, (C₅-C₈)Cycloalkyl, Aryl, wobei Aryl substituiert sein kann mit F, Cl, Br, J, -OH, -O(C₁-C₃)Alkyl, -NH₂, -NH(C₁-C₃)Alkyl, -N[(C₁-C₃)Alkyl]₂, - C(O)OH, -C(O)O(C₁-C₃)Alkyl, -C(O)NH₂, -C(O)NH(C₁-C₃)Alkyl, -C(O)N[(C₁-C₃)Alkyl]₂, -SO₂NH₂, -SO₂NH(C₁-C₃)Alkyl, -SO₂N[(C₁-C₃)Alkyl]₂, -CN, (C₁-C₁₂)Alkyl und (C₅-C₈)Cycloalkyl, bedeutet,
und 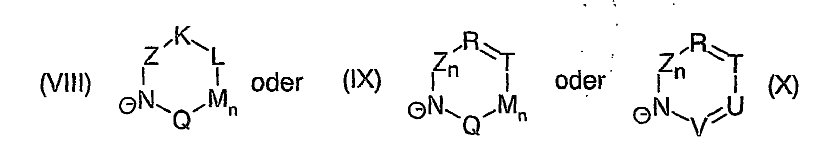 oder 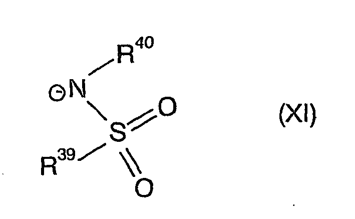 als Anion dargestellt wird
und die Symbole, Substituenten und Indices folgende Bedeutung haben,
- Z =: C=O, C=S, S=O, SO₂ oder C=NR²⁰
- K =: O, S, NR²¹ oder CR²²R²³
- L =: NR²⁴ oder CR²⁵R²⁶
- n =: 0 oder 1
- M =: O, C=O, NR²⁷ oder CR²⁸R²⁹
- Q =: O, S, NR³⁰, CR³¹R³², C=O, C=S, S=O, SO₂ oder C=NR³⁴
- R =: CR³⁵ oder N
- T =: CR³⁶ oder N
- U =: CR³⁷ oder N
- V =: CR³⁸ oder N
wobei R²⁰ bis R³² und R³⁴ bis R³⁸ unabhängig voneinander H, (C₁-C₆)Alkyl, Aryl oder Heteroaryl bedeuten und jeweils zwei Alkylreste gemeinsam auch einen Cycloalkylenrest von maximal 6 C-Bausteinen im Ring bilden können, der wiederum substituiert sein kann mit F, Cl, Br, I, CF₃, NO₂, COO(C₁-C₆)Alkyl, CON[(C₁-C₆)Akyl]₂, Cycloalkyl, (C₁-C₁₀)-Alkyl, (C₂-C₆)Alkenyl, O-(C₁-C₆)Alkyl, O-CO-(C₁-C₆)Alkyl, O-CO-(C₁-C₆)Alkylen-aryl, SO₂N[(C₁-C₆)Alkyl]₂, S-(C₁-C₆)Alkyl, S-(CH₂-)ₙAryl, SO-(C₁-C₆)Alkyl, SO-(CH₂-)ₙAryl, SO₂-(C₁-C₆)Alkyl, SO₂-(CH₂-)ₙAryl, SO₂-N((C₁-C₆)-Alkyl)(CH₂-)ₙAryl, oder SO₂-N((CH₂-)ₙAryl)_{2,} wobei n = 0 bis 6 sein kann und der Arylrest bis zu zweifach mit F, Cl, Br, CF₃, SF₅, NO₂, OCF₃, O-(C₁-C₆)Alkyl oder (C₁-C₆)Alkyl substituiert sein kann; oder mit N((C₁-C₆)Alkyl)₂, NH-CO-NH-(C₁-C₆)Alkyl), NH-CO-NH-Aryl, N[(C₁-C₆)Alkyl]-CO-(C₁-C₆)Alkyl, N[(C₁-C₆)Alkyl]-COO-(C₁-C₆)Alkyl, N[(C₁-C₆)Alkyl]-CO-Aryl, N[(C₁-C₆)Alkyl]-COO-Aryl, N[(C₁-C₆)Alkyl]-CO-N((C₁-C₆)Alkyl)₂, N[(C₁-C₆)Alkyl]-CO-N((C₁-C₆)Akyl)-Aryl, N[(C₁-C₆)Alkyl]-CO-N(Aryl)₂, N(Aryl)-CO-(C₁-C₆)Alkyl, N(Aryl)-COO-(C₁-C₆)Alkyl, N(Aryl)-CO-Aryl, N(Aryl)-COO-Aryl, N(Aryl)-CO-N((C₁-C₆)Alkyl)₂, N(Aryl)-CO-N[(C₁-C₆)Alkyl]-Aryl, N(Aryl)-CO-N(Aryl)₂, Aryl, O-(CH₂-)ₙAryl, wobei n = 0 bis 6 sein kann, wobei der Aryl-Rest 1 bis 3-fach substituiert sein kann mit F, Cl, Br, I, CF₃, NO₂, OCF₃, O-(C₁-C₆)Alkyl, (C₁-C₆)Alkyl, N((C₁-C₆)Alkyl)₂, SF₅, SO₂-CH₃ oder COO-(C₁-C₆)Alkyl
und wobei R³⁹ und R⁴⁰ unabhängig voneinander (C₁-C₆)Alkyl, wobei ein oder mehrere nicht benachbarte C-Atome durch NH oder C=O ersetzt sein können, (C₁-C₆)-Perfluoralkyl, Aryl oder Heteroaryl bedeuten, oder R³⁹ und R⁴⁰ bilden zusammen ein 1,8-Naphthyl oder 1,7,7-Trimethyl-bicyclo[2.2.1]heptanyl, R⁴⁰ kann auch H sein,
oder wobei in diesem Zyklisierungskatalysator das Kation dem der allgemeinen Formel (XII) entspricht und das Anion R⁴¹O⁻ ist, oder wobei in diesem Zyklisierungskatalysator das Kation dem der allgemeinen Formel (XII) entspricht und das Anion R⁴²COO- ist, oder wobei in diesem Zyklisierungskatalysator das Kation der allgemeinen Formel (XII) entspricht, und das Anion Cl⁻, Br⁻ oder I⁻ ist und diese mit Ag₂O kombiniert werden.

Unter Aryl ist dabei ein aromatischer Kohlenwasserstoffrest zu verstehen, der 6 bis 14 C-Atome aufweist, z.B. Phenyl, Naphthyl-, Biphenyl-, Tetrahydronaphthyl-, alpha- oder beta-Tetralon-, Indanyl- oder Indan-1-on-ylrest und bevorzugt unsubstituiert ist, aber auch substituiert sein kann.

Typische Substituenten sind dabei z.B. F, Cl, Br, I, CF₃, NO₂, COO(C₁-C₆)Alkyl, CON[(C₁-C₆)Alkyl]₂, Cycloalkyl, (C₁-C₁₀)Alkyl, (C₂-C₆)Alkenyl, O-(C₁-C₆)Alkyl, O-CO-(C₁-C₆)Alkyl, O-CO-(C₁-C₆)Alkylen-aryl, SO₂N[(C₁-C₆)Alkyl]₂, S-(C₁-C₆)Alkyl, S-(CH₂-)ₙAryl, SO-(C₁-C₆)Alkyl, SO-(CH₂-)ₙAryl, SO₂-(C₁-C₆)Alkyl, SO₂-(CH₂-)ₙAryl, SO₂-N((C₁-C₆)Alkyl)(CH₂-)ₙAryl oder SO₂-N((CH₂-)ₙAryl)₂, wobei n = 0 bis 6 sein kann und der Arylrest bis zu zweifach mit F, Cl, Br, CF₃, SF₅, NO₂, OCF₃, O-(C₁-C₆)Alkyl oder (C₁-C₆)-Alkyl substituiert sein kann; oder auch N((C₁-C₆)Alkyl)₂, NH-CO-NH-(C₁-C₆)Alkyl), NH-CO-NH-Aryl, N[(C₁-C₆)Alkyl]-CO-(C₁-C₆)Alkyl, N[(C₁-C₆)Alkyl]-COO-(C₁-C₆)Alkyl, N[(C₁-C₆)Alkyl]-CO-Aryl, N[(C₁-C₆)Alkyl]-COO-Aryl, N[(C₁-C₆)Alkyl]-CO-N((C₁-C₆)Alkyl)₂, N[(C₁-C₆)Alkyl]-CO-N((C₁-C₆)Alkyl)-Aryl, N[(C₁-C₆)Alkyl]-CO-N(Aryl)₂, N(Aryl)-CO-(C₁-C₆)Alkyl, N(Aryl)-COO-(C₁-C₆)Alkyl, N(Aryl)-CO-Aryl, N(Aryl)-COO-Aryl, N(Aryl)-CO-N((C₁-C₆)Alkyl)₂, N(Aryl)-CO-N[(C₁-C₆)Alkyl]-Aryl, N(Aryl)-CO-N(Aryl)₂, Aryl, O-(CH₂-)ₙAryl, wobei n = 0 bis 6 sein kann, wobei der Aryl-Rest 1 bis 3-fach substituiert sein kann mit F, Cl, Br, I, CF₃, NO₂, OCF₃, O-(C₁-C₆)Alkyl, (C₁-C₆)Alkyl, N((C₁-C₆)Alkyl)₂, SF₅, SO₂-CH₃ oder COO-(C₁-C₆)Alkyl

Unter Heteroaryl werden dabei aromatische Ringe und Ringsysteme verstanden, die außer Kohlenstoff noch Heteroatome wie Stickstoff, Sauerstoff oder Schwefel enthalten. Ferner gehören auch Ringsysteme zu dieser Definition, worin der heterozyklische Rest mit Benzolkernen kondensiert ist. Die Ringe sind bevorzugt 3- bis 7-gliedrig.

Unter einem Alkylrest wird eine geradkettige oder verzweigte Kohlenwasserstoffkette mit einem oder mehreren Kohlenstoffen, bevorzugt 1 bis 8 Kohlenstoffen, verstanden, z.B. Methyl, Ethyl, Propyl, Butyl, Hexyl, Isopropyl, Isobutyl, Neopentyl, tert.-Butyl, Hexyl.

Unter einem Cycloalkylrest wird ein Rest verstanden, der aus einem oder mehrere Ringe enthaltenden Ringsystem besteht, welches gesättigt oder partiell ungesättigt (mit einer oder zwei Doppelbindungen) vorliegt, das ausschließlich aus Kohlenstoffatomen aufgebaut ist, z.B. Cyclopropyl, Cyclopentyl, Cyclopentenyl, Cyclohexyl oder Adamantyl. Die Ringe sind bevorzugt 3- bis 7-gliedrig. Als mögliche Substituenten sind die vorstehend genannten typisch.

Eine bevorzugte Ausführungsform des erfindungsgemäßen Verfahrens geht aus von dem bekannten Verfahren zur Herstellung von 1,4-Diphenylazetidinon-Derivaten der allgemeinen Formel (I) 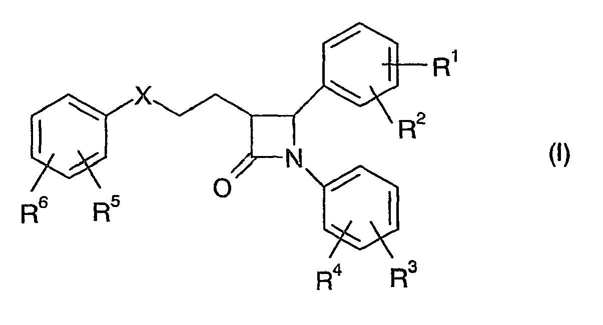 in der die Symbole, Substituenten und Indices folgende Bedeutung haben,
- X =: CH₂, CHOH, CO oder CHOCOR¹¹
- R¹, R² =: unabhängig voneinander H, OH, OCF₃, oder O-(C₁-C₆)Alkyl, O-(C₃-C₇)Cycloalkyl, O-COR¹¹, CN, CH₂NHR⁷, CH₂NR⁷R⁸, NR⁷R⁸, COR¹⁴, F oder Cl
- R³, R⁴ =: unabhängig voneinander H, F, Cl, OH, OCF₃, O-(C₁-C₆)Alkyl, O-(C₃-C₇)Cycoalkyl, O-COR¹¹, CN, CH₂NHR⁷, CH₂NR⁷R⁸, NR⁷R⁸, COR¹⁴ oder (C₁-C₆)Alkyl
- R⁵, R⁶ =: unabhängig voneinander H, F, Cl, (C₁-C₆)Alkyl, CF₃ oder OCF₃
- R⁷ =: H, C(=O)-Y(-CH₂)ₖ-Y-C(=O)R⁹ oder C(=O)-Y(-CH₂)ₖ-NHR¹⁰
- k =: 2 bis 16
- Y =: Einfachbindung oder NR¹³
- R⁸ =: H, (C₁-C₆)Alkyl, oder (C₃-C₇)Cycloalkyl
- R⁹ =: OH oder NHCH₂[-CH(OH)]ₘ-CH₂OH oder eine in geeigneter Weise geschütze Form davon
- R¹⁰ =: H, C(=O)[-CH(OH)]ₘ-CH₂OH oder eine in geeigneter Weise geschützte Form davon
- m =: 0 bis 5
- R¹¹ =: H, (C₁-C₆)Alkyl, (C₃-C₇)Cycloalkyl, (substituiertes)Phenyl oder OR¹²
- R¹² =: (C₁-C₆)Alkyl oder (C₃-C₇)Cycloalkyl
- R¹³ =: (C₁-C₆)Alkyl oder (C₃-C₇)Cycloalkyl, Aryl oder Heteroaryl
- R¹⁴ =: OH, OR¹², NR¹³(-CH₂)ₙ-Y-C(=O)R⁹ oder NR¹³(-CH₂)ₙ-NHR¹⁰
in Gegenwart eines Silylierungsmittels und eines Zyklisierungskatalysators.

Das erfindungsgemäße Verfahren ist dann dadurch gekennzeichnet, dass Verbindungen der allgemeinen Formel (IV) 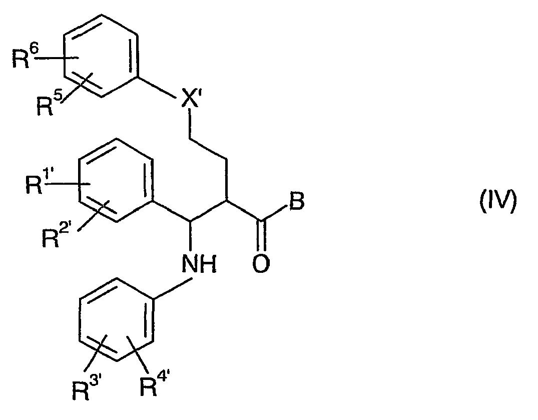 in der die Symbole, Substituenten und Indices - soweit nicht vorstehend definiert - folgende Bedeutung haben,
- X' =: X, CHOSi(Alkyl)ₒ(Aryl)ₚ mit o, p = 1 bis 3 und o + p = 3, C(OAlkyl)₂ oder zyklisches Ketal wie C[O(-CH₂)_{q}-O] mit q = 2, 3
- R^{1'}, R^{2'} =: R¹, R² und O-Schutzgruppe
- R^{3'}, R^{4'} =: R³, R⁴, CH₂NHCO₂CH₂(C₆H₅), CH₂N[Si(Alkyl)ₒ(Aryl)ₚ]CO₂CH₂(C₆H₅), CH₂NHCO₂-tert.Bu, CH₂N[Si(Alkyl)ₒ(Phenyl)ₚ]CO₂-tert.Bu, CH₂NHC(C₆H₅)₃, CH₂N=C(C₆H₅)₂ oder CH₂N=CH[C₆H₄(R⁶)] 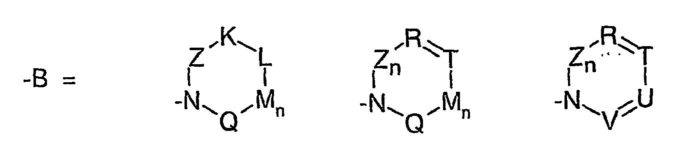
oder 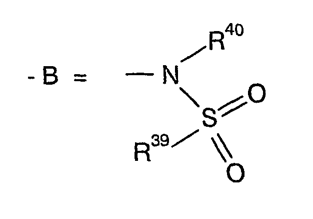 zu (Vor-)Produkten der allgemeinen Formel (V) zyklisiert werden, die zur Verbindung (I) entschützt werden können, 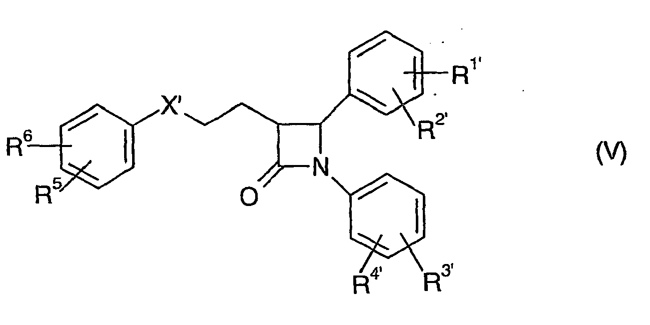 wobei der Zyklisierungskatalysator durch eine der nachstehenden allgemeinen Formeln 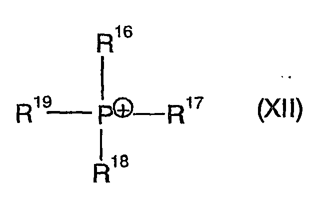 als Kation, wobei R¹⁶, R¹⁷, R¹⁸, R¹⁹ unabhängig voneinander Aryl, (C₁-C₁₅)Alkyl - insbesondere (C₁-C₁₀)Alkyl-, Benzyl-, insbesondere Butyl bedeuten,
R⁴¹ Aryl, (C₁-C₁₅)Alkyl - insbesondere (C₁-C₁₀)Alkyl-, Benzyl bedeutet,
R⁴² (C₁-C₁₅)Alkyl - insbesondere (C₁-C₁₀)Alkyl-, Benzyl, (C₅-C₈)Cycloalkyl, Aryl, wobei Aryl substituiert sein kann mit F, Cl, Br, J, -OH, -O(C₁-C₃)Alkyl, -NH₂, -NH(C₁-C₃)Alkyl, -N[(C₁-C₃)Alkyl]₂, -C(O)OH, -C(O)O(C₁-C₃)Alkyl, -C(O)NH₂, -C(O)NH(C₁₋ C₃)Alkyl, -C(O)N[(C₁-C₃)Alkyl]₂, -SO₂NH₂, -SO₂NH(C₁-C₃)Alkyl, -SO₂N[(C₁-C₃)Alkyl]₂, -CN, (C₁-C₁₂)Alkyl und (C₅-C₈)Cycloalkyl, bedeutet,
und 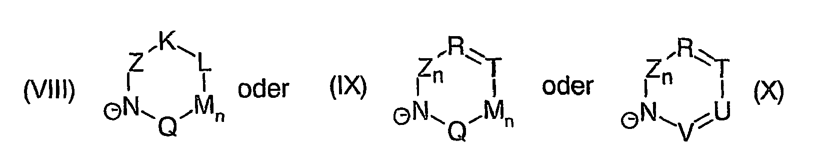 oder 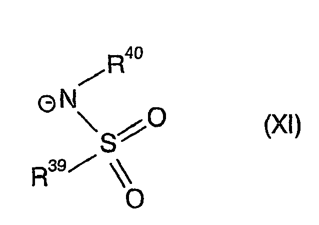 als Anion dargestellt wird.
oder wobei in diesem Zyklisierungskatalysator das Kation dem der allgemeinen Formel (XII) entspricht und das Anion R⁴¹O⁻ ist, oder wobei in diesem Zyklisierungskatalysator das Kation dem der allgemeinen Formel (XII) entspricht und das Anion R⁴²COO⁻ ist oder wobei in diesem Zyklisierungskatalysator das Kation der allgemeinen Formel (XII) entspricht und das Anion Cl⁻, Br⁻ oder I⁻ ist und diese mit Ag₂O kombiniert werden.

In einer besonders bevorzugten Ausführungsform ist das erfindungsgemäße Verfahren dadurch gekennzeichnet, dass Verbindungen der allgemeinen Formel (II) 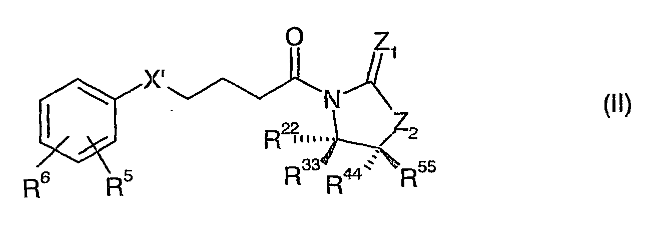 mit Iminen der allgemeinen Formel (III) 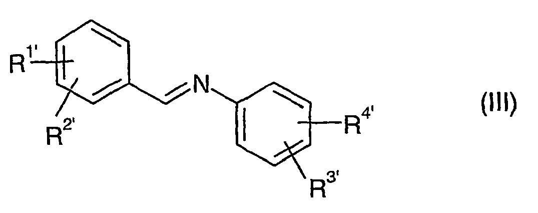 zu Zwischenstufen der allgemeinen Formel (IV'), 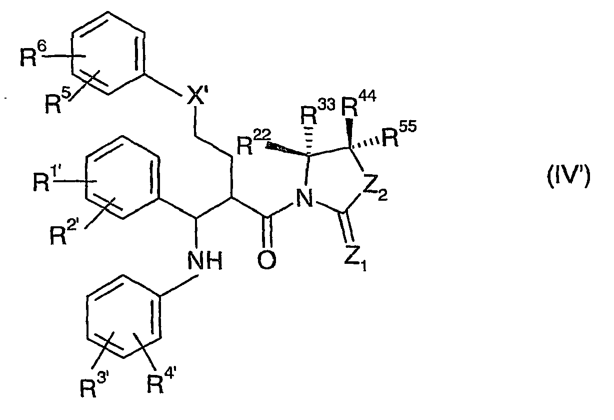 umgesetzt werden, die dann zu (Vor)Produkten der allgemeinen Formel (V) zyklisiert werden, die zur Verbindung (I) entschützt werden können, 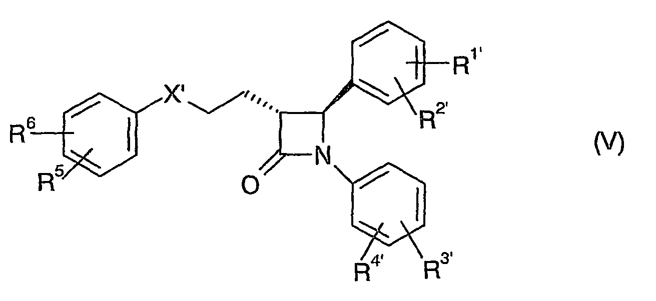 in der die Symbole, Substituenten und Indices - soweit nicht vorstehend definiert - folgende Bedeutung haben,
- X' =: X, CHOSi(Alkyl)ₒ(Aryl)ₚ mit o, p = 1 bis 3 und o + p = 3, C(OAlkyl)₂ oder zyklisches Ketal wie C[O(-CH₂)_{q}-O] mit q = 2, 3
- R²², R³³, R⁴⁴, R⁵⁵ =: unabhängig voneinander H, Aryl oder (C₁-C₁₀)Alkyl
- Z₁, Z₂ =: unabhängig voneinander O, NH, NR¹⁵, oder S
- R¹⁵ =: Aryl oder (C₁-C₁₀)Alkyl,
- R^{1'}, R^{2'} =: R¹, R² und O-Schutzgruppe
- R^{3'}, R^{4'} =: R³, R⁴, CH₂NHCO₂CH₂(C₆H₅), CH₂N[Si(Alkyl)ₒ(Aryl)ₚ]CO₂CH₂(C₆H₅), CH₂NHCO₂-tert.Bu, CH₂N[Si(Alkyl)ₒ(Phenyl)ₚ]CO₂-tert.Bu, CH₂NHC(C₆H₅)₃, CH₂N=C(C_{6H5})₂ oder CH₂N=CH[C₆H₄(R⁶)]
wobei der Zyklisierungskatalysator durch eine der allgemeinen Formeln (VIa) bis (VII) dargestellt wird 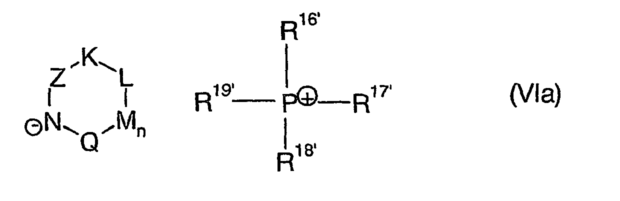 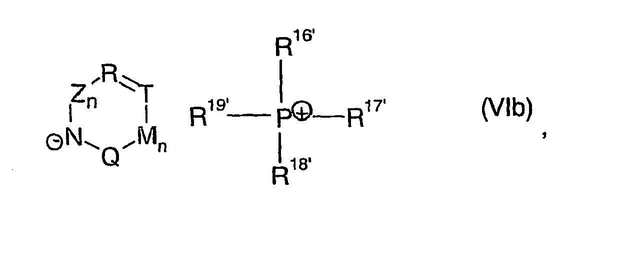 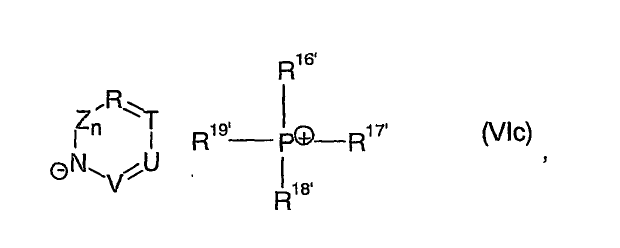 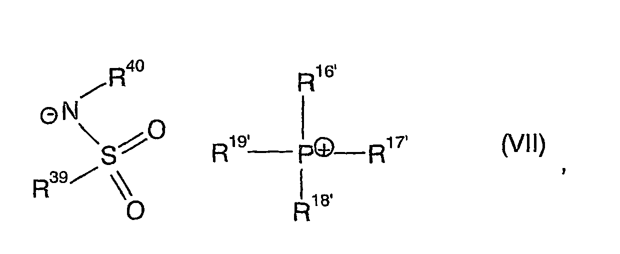 dabei sind R^{16'}, R^{17'}, R^{18'}, R^{19'} unabhängig voneinander Aryl, (C₁-C₁₅)Alkyl.

In einer weiteren besonders bevorzugten Ausführungsform ist das erfindungsgemäße Verfahren dadurch gekennzeichnet, dass Verbindungen der allgemeinen Formel (II) 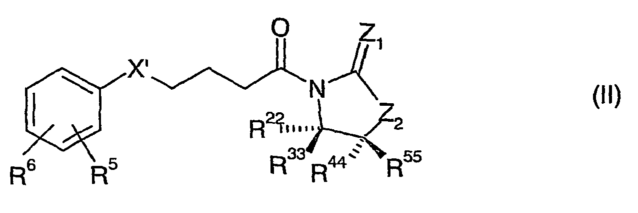 mit Iminen der allgemeinen Formel (III) 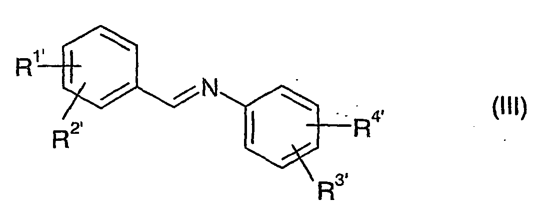 zu Zwischenstufen der allgemeinen Formel (IV'), 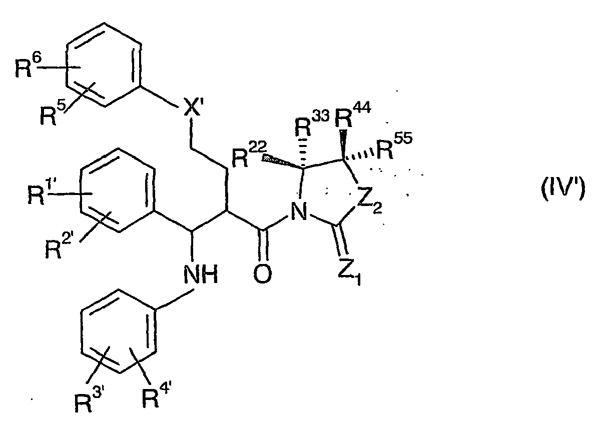 umgesetzt werden, die dann zu (Vor)Produkten der allgemeinen Formel (V) zyklisiert werden, die zur Verbindung (I) entschützt werden können, 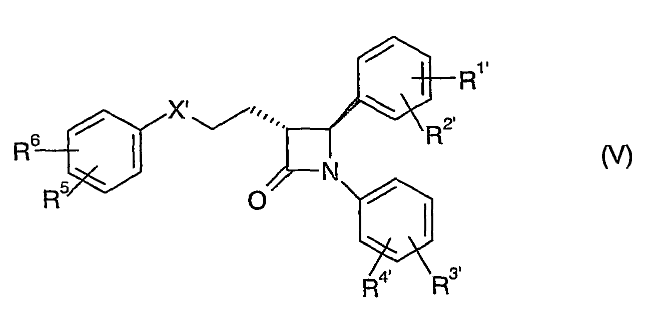 in der die Symbole, Substituenten und Indices- soweit nicht vorstehend definiert - folgende Bedeutung haben,
- X' =: X, CHOSi(Alkyl)ₒ(Aryl)_{P} mit o, p = 1 bis 3 und o + p = 3, C(OAlkyl)₂ oder zyklisches Ketal wie C[O(-CH₂)_{q}-O] mit q = 2, 3
- R²², R³³, R⁴⁴, R⁵⁵ =: unabhängig voneinander H, Aryl oder (C₁-C₁₀)Alkyl
- Z₁, Z₂ =: unabhängig voneinander O, NH, NR¹⁵, oder S
- R¹⁵ =: Aryl oder (C₁-C₁₀)Alkyl,
- R^{1'}, R^{2'} =: R¹, R² und O-Schutzgruppe
- R^{3'}, R^{4'}=: R³, R⁴, CH₂NHCO₂CH₂(C₆H₅), CH₂N[Si(Alkyl)1ₒ(Aryl)ₚ]CO₂CH₂(C₆H₅), CH₂NHCO₂-tert.Bu, CH₂N(Si(Alkyl)ₒ(Phenyl)ₚ]CO₂-tert.Bu, CH₂NHC(C₆H₅)₃, CH₂N=C(C₆H₅)₂ oder CH₂N=CH[C₆H₄(R⁶)]
wobei im Zyklisierungskatalysator das Kation dem der allgemeinen Formel (XII) entspricht und das Anion R⁴¹O⁻ ist.

In einer weiteren besonders bevorzugten Ausführungsform ist das erfindungsgemäße Verfahren dadurch gekennzeichnet, dass Verbindungen der allgemeinen Formel (II) 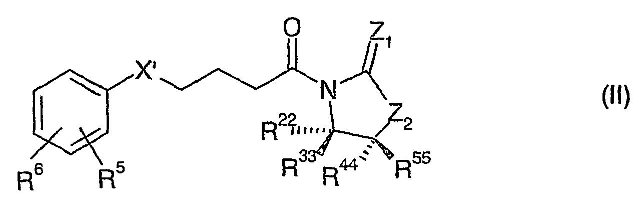 mit Iminen der allgemeinen Formel (III) 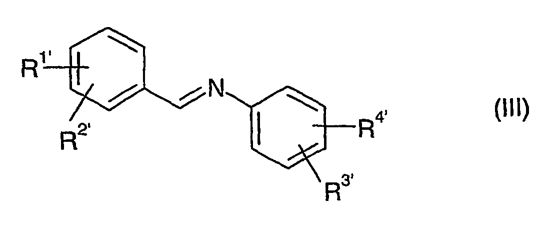 zu Zwischenstufen der allgemeinen Formel (IV'), 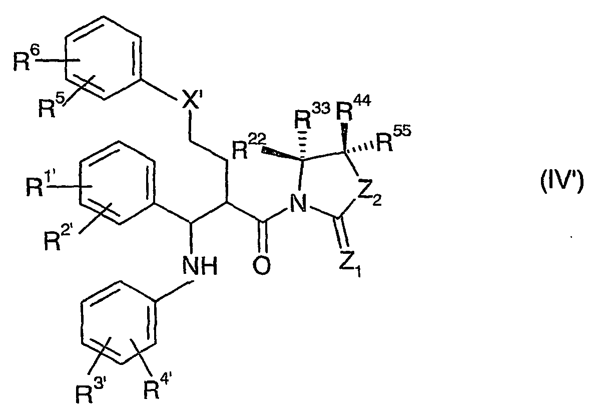 umgesetzt werden, die dann zu (Vor)Produkten der allgemeinen Formel (V) zyklisiert werden, die zur Verbindung (I) entschützt werden können, 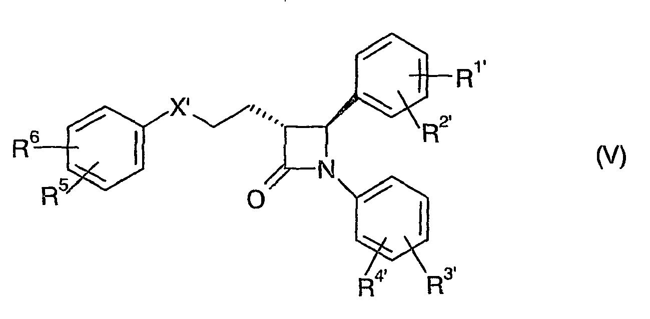 in der die Symbole, Substituenten und Indices - soweit nicht vorstehend definiert - folgende Bedeutung haben,
- X' =: X, CHOSi(Alkyl)ₒ(Aryl)ₚ mit o, p = 1 bis 3 und o + p = 3, C(OAlkyl)₂ oder zyklisches Ketal wie C[O(-CH₂)_{q}-O] mit q = 2, 3
- R²², R³³, R⁴⁴, R⁵⁵ =: unabhängig voneinander H, Aryl oder (C₁-C₁₀)Alkyl

- Z₁, Z₂ =: unabhängig voneinander O, NH, NR¹⁵, oder S
- R¹⁵ =: Aryl oder (C₁-C₁₀)Alkyl,
- R^{1'}, R^{2'} =: R¹, R² und O-Schutzgruppe
- R^{3'}, R^{4'} =: R³, R⁴, CH₂NHCO₂CH₂(C₆H₅), CH₂N[Si(Alkyl)ₒ(Aryl)ₚ]CO₂CH₂(C₆H₅), CH₂NHCO₂-tert.Bu, CH₂N[Si(Alkyl)ₒ(Phenyl)ₚ]CO₂-tert.Bu, CH₂NHC(C₆H₅)₃, CH₂N=C(C₆H₅)₂ oder CH₂N=CH[C₆H₄(R⁶)]
wobei im Zyklisierungskatalysator das Kation dem der allgemeinen Formel (XII) entspricht und das Anion R⁴²COO- ist.

Insbesondere bevorzugt sind Zyklisierungskatalysatoren mit einem Anion, das sich ableitet von
Oxazolidin-2-on
4-Benzyl-oxazolidin-2-on
4-Phenyl-oxazolidin-2-on
4-Isopropyl-oxazolidin-2-on
4-tert-Butyl-oxazolidin-2-on
4-Isopropyl-5,5-dimethyl-oxazolidin-2-on
4-Benzyl-5,5-dimethyl-oxazolidin-2-on
4-Phenyl-5,5-dimethyl-oxazolidin-2-on
4-Isopropyl-5,5-dimethyl-oxazolidin-2-on
4-tert-Butyl-5,5-dimethyl-oxazolidin-2-on
4-Methyl-5-phenyl-oxazolidin-2-on
cis-4,5-Diphenyl-oxazolodin-2-on
4-Isopropyl-5,5-diphenyl-oxazolidin-2-on
1-Methyl-4-methylen-imidazolidin-2-on
Imidazol
Phthalimid
2,10-Camphersultam
1-Phenyl-3-pyrazolidinon,
oder das ein Alkoxid ist
oder das ein Carboxylat ist
in Kombination mit Tetraethylphosphonium, Tetrabutylphosphonium oder Tetraoctylphosphonium als Kation.

Die erfindungsgemäß herstellbaren Diphenylazetidinon-Verbindungen werden in der Praxis häufig als pharmazeutisch verträgliches Salz eingesetzt, denn diese sind aufgrund ihrer höheren Wasserlöslichkeit gegenüber den Ausgangs- beziehungsweise Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäß herstellbaren Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie zum Beispiel Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluol sulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Die Verbindungen der allgemeinen Formel (1) und deren pharmazeutisch verträgliche Salze und physiologisch funktionellen Derivate stellen ideale Arzneimittel zur Behandlung von Lipidstoffwechselstörungen, insbesondere von Hyperlipidämie dar. Die Verbindungen der allgemeinen Formel (I) eignen sich ebenfalls zur Beeinflussung des Serumcholesterinspiegels sowie zur Prävention und Behandlung arteriosklerotischer Erscheinungen.

Zu weiteren die Verbindungen selbst betreffenden Einzelheiten und zu ihrer Verarbeitung, Kombination mit anderen Wirkstoffen etc. wird ausdrücklich auf die WO 02/50027 verwiesen.

In der nachfolgenden Tabelle sind typische Beispiele für den Rest -B der allgemeinen Formel (IV) beziehungsweise in anionischer Form für die allgemeinen Formeln (VIII) bis (XI) aufgeführt, wobei jeweils in den Tabellen dann das "H" am "HN" im Ring beziehungsweise am Molekül überflüssig ist, um den Rest -B beziehungsweise das zugehörige Anion B^{Θ} zu ergeben.

Beispiele für H-B beziehungsweise H^{⊕}B^{⊖} in den allgemeinen Formeln (II), (IV) u. (VIII) bis (XI) sind

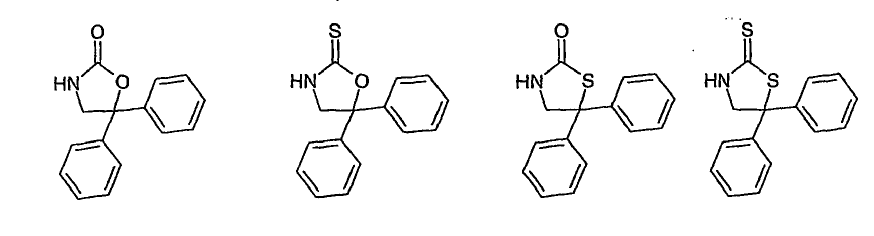

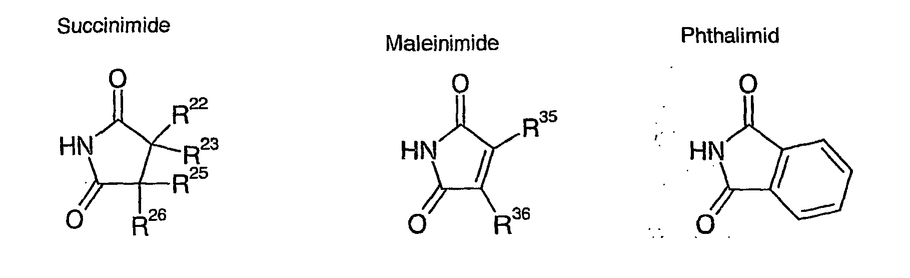

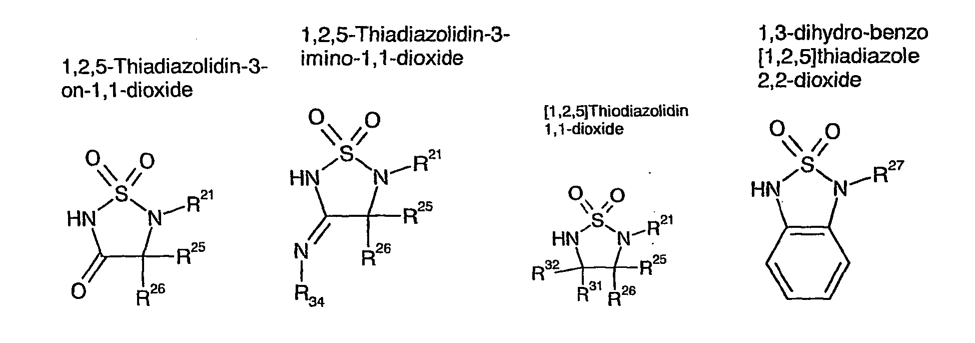

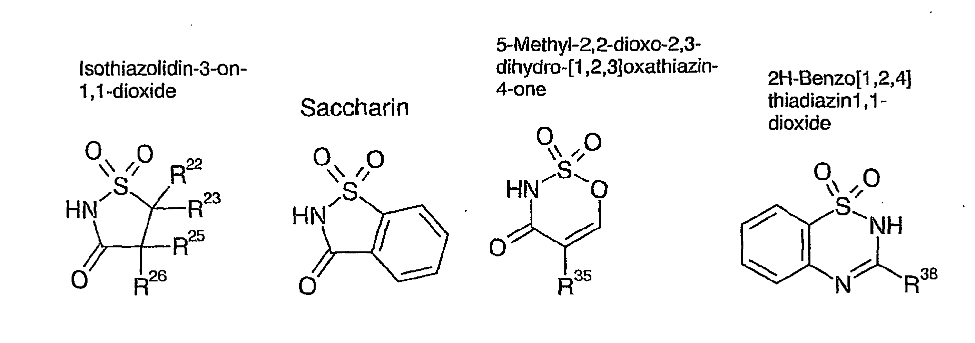

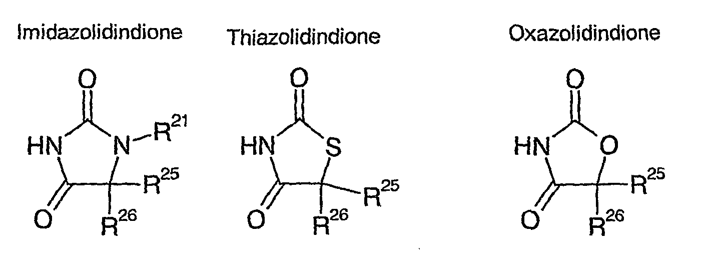

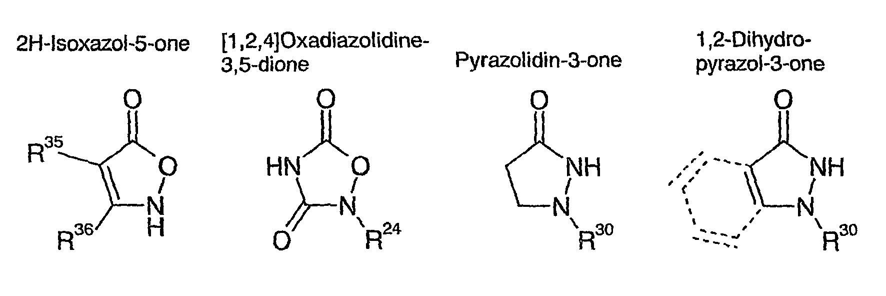

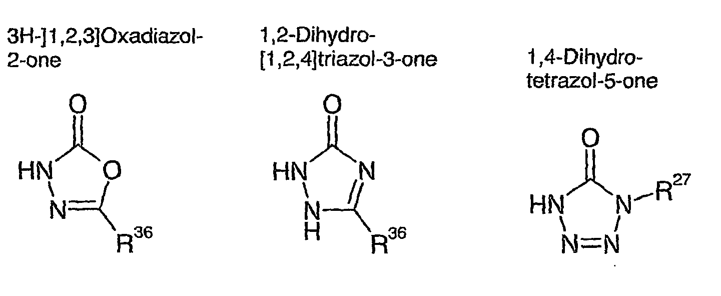

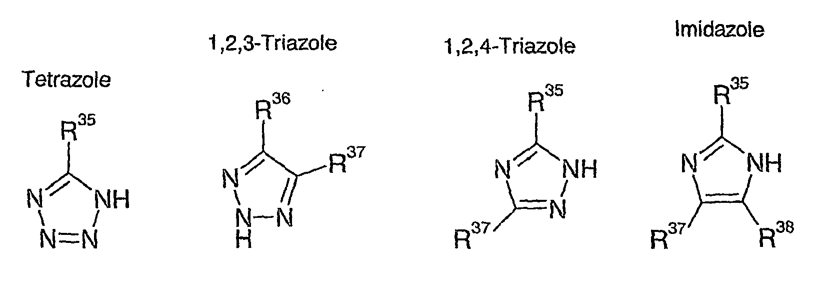

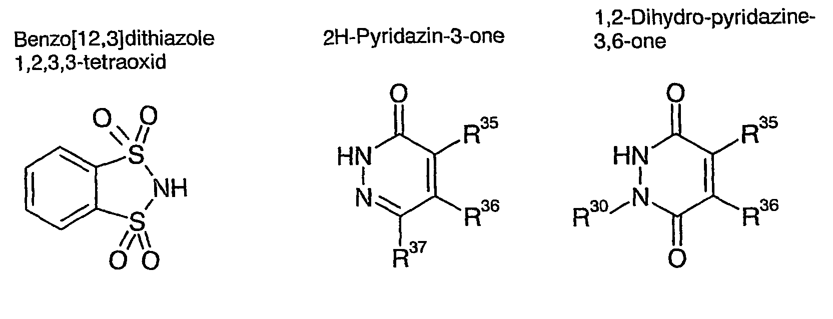

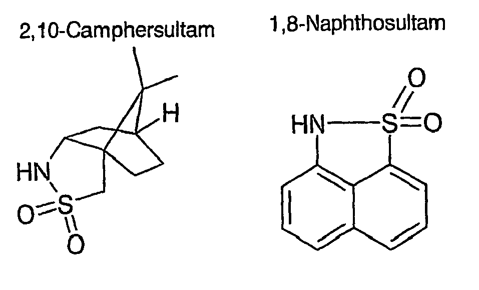

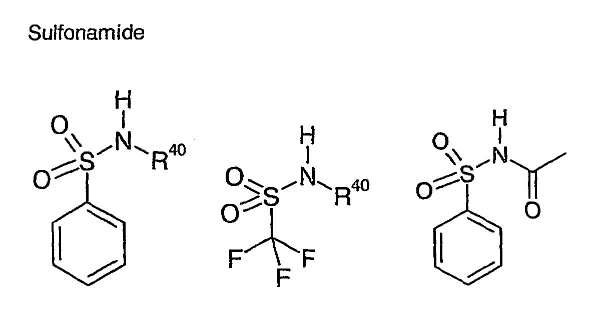

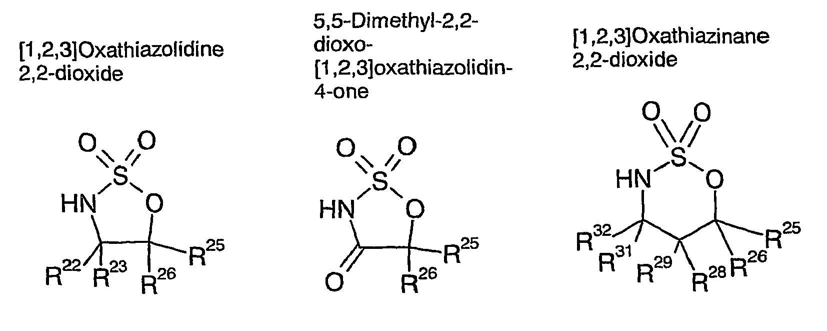

Die eingangs beschriebenen Azetidinone der allgemeinen Formel (V) werden insbesondere erhalten, indem die Verbindungen der allgemeinen Formel (IV) in einem organischen Lösungsmittel wie z.B. Hexan, Heptan, Toluol, Chlorbenzol, Diisopropylether, Ethylacetat, Dimethoxyethan, Dichlormethan oder tert-Butylmethylether, vorzugsweise aber Toluol, Diisopropylether oder tert-Butylmethylether suspendiert und anschließend mit einem milden Silylierungsmittel, wie z.B. 2-6 Äquivalente N,O-Bistrimethylsilylacetamid, vorzugsweise aber 4-6 Äquivalente, besonders bevorzugt 5,7 Äquivalente und einer katalytischen Menge von 1-25 Mol-%, vorzugsweise 5-15 Mol-%, besonders bevorzugt 5 Mol-% eines der vorgenannt beschriebenen Katalysatoren versetzt werden. Die Reihenfolge der Zugabe der Reagentien spielt keine Rolle. Das Gemisch wird auf 20°C bis 100°C, vorzugsweise 40°C bis 60°C erwärmt und etwa 1 bis 20 Stunden, vorzugsweise 6 Stunden gerührt, oder bis die Umsetzung vollständig abgeschlossen ist. Die Verbindungen der allgemeinen Formel (V) werden anschließend durch übliche Extraktionsmethoden isoliert.

Sofern die Verbindungen der allgemeinen Formel (V) noch zu Verbindungen der allgemeinen Formel (I) umgesetzt werden, in denen bei R¹, R³ = H und R², R⁴ = CH₂NHR₇ bedeutet (R ≠ H), so werden Amine der allgemeinen Formel (V), d.h. solche Verbindungen, in denen R^{2'}, R^{4'} nach Entschützung R², R⁴ und zwar CH₂NHR₇ mit R = H bedeutet, mit Verbindungen der nachstehenden allgemeinen Formeln (XIII) oder (XIV) 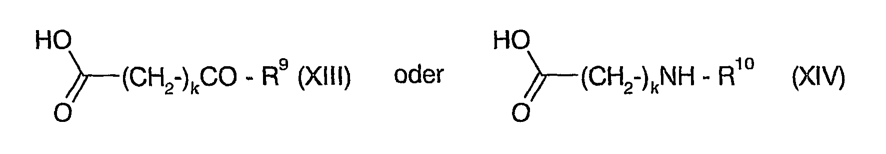 zu den gewünschten Verbindungen nach vorheriger Schutzgruppenabspaltung umgesetzt (unter Peptidkupplung). Andere Verbindungen der allgemeinen Formel (I) entstehen durch einfache Entschützung der entsprechenden Verbindungen der allgemeinen Formel (V) ohne weitere Umsetzungen.

Zur detaillierten Charakterisierung der Verfahrensbedingungen wird auf die nachfolgenden Beispiele und den einleitend zitierten Stand der Technik verwiesen, dies gilt auch für die üblicherweise eingesetzten Silylierungsmittel.

Die Erfindung wird in den nachfolgenden Beispielen näher erläutert.

### Beispiele

### Beispiel 1

(*n*Bu)₄P^{+ -}O-CH₃

### Tetrabutylphosphoniummethoxid

Bei Raumtemperatur wird Kaliummethoxylat (475,5 mg) unter einer Argonatmosphäre in Methanol (7 ml) suspendiert. Dazu wird eine methanolische Lösung (3 ml) von Tetrabutylphosphoniumchlorid (2 g) gegeben. Es wird 2 Stunden bei Raumtemperatur gerührt, unter Argon über einen Spritzenfilter filtriert und das Lösemittel entfernt. Der Rückstand wird gewogen und in Tetrahydrofuran (THF) (5 ml) aufgenommen. Es wird eine 1,35 molare Lösung von Tetrabutylphosphoniummethoxid erhalten.

### Beispiel 2

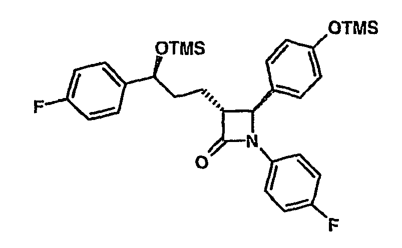

### Zyklisierung von 3-{5-(4-Fluorophenyl)-2-[(4-fluorophenylamino)-(4-trimethylsilanyl-oxyphenyl)-methyl]-5-trimethylsilanyloxypentanoyl}-4-phenyloxazolidin-2-on mit der Verbindung aus Beispiel 1

3-{5-(4-Fluorophenyl)-2-[(4-fluorophenylamino)-(4-trimethylsilanyloxyphenyl)-methyl]-5-trimethylsilanyloxypentanoyl}-4-phenyloxazolidin-2-on (10 mg) wird unter Argon und bei Eiskühlung in Methyl-t-butyl-Ether (MTB-Ether) (1 ml) suspendiert. N,O-Bistrimethylsilylacteamid (20,75 µl) wird zugegeben, gefolgt von Tetrabutylphosphoniummethoxid (20,65 µl; 1,35 M in THF). Es wird eine Stunde lang bei Raumtemperatur gerührt. Mittels Dünnschichtchromatographie und LC/MS-Vergleich (Liquid-Chromatography/Mass-Spectrometry) wird eine Umsetzung zu 1-(4-Fluorophenyl)-3-[3-(4-fluorophenyl)-3-trimethylsilanyl-oxypropyl]-4-(4-trimethylsilanyloxy-phenyl)-azetidin-2-on festgestellt (M+H,-TMS,-HOTMS: 392).

### Beispiel 3

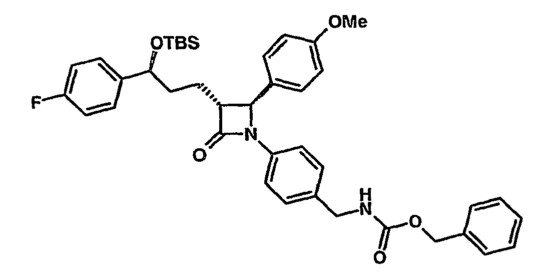

### Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäurebenzylester mit der Verbindung aus Beispiel 1

Unter Eiskühlung und in einer Argonatmosphäre wird {4-[5-(tert-Butyldimethylsilanyloxy)-5-(4-fluorophenyl)-1-(4-methoxy-phenyl)-2-(2-oxo-4-phenyloxazolidine-3-carbonyl)-pentyl-amino]-benzyl}-carbamylsäurebenzylester (10 mg) in MTB-Ether suspendiert (1 ml). N,O-Bistrimethylsilylacteamid (17,5 µl) wird zugegeben, gefolgt von Tetrabutylphosphoniummethoxid (23,6 µl; 1 M in THF). Es wird zwei Stunden lang bei Raumtemperatur gerührt. Die Reaktion wird mit Wasser gequencht und die wässrige Phase mit Ethylacetat extrahiert. Mittels Dünnschichtchromatographie und LC/MS-Vergleich wird eine Umsetzung zu {4-[3-[3-(tert-Butyldimethylsilanyloxy)-3-(4-fluorophenyl)-propyl]-2-(4-methoxyphenyl)-4-oxo-azetidin-1-yl]-benzyl}-carbamylsäurebenzylester festgestellt. ¹H-NMR (d⁶-DMSO) -0.18 (s, 3 H), 0.02 (s, 3 H), 0.85 (s, 9 H), 1.75 (bs, 4 H), 3.05 (bs, 1 H), 3.7 (s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10 H), 7.73 (t, 1 H).

### Beispiel 4

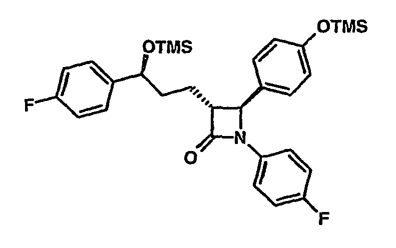

### Zyklisierung von 3-{5-(4-Fluorophenyl)-2-[(4-fluorophenylamino)-(4-trimethylsilanyl-oxyphenyl)-methyl]-5-trimethylsilanyloxypentanoyl}-4-phenyloxazolidin-2-on mit Tetrabutylphosphoniumchlorid und Silber(I)oxid

3-{5-(4-Fluorophenyl)-2-[(4-fluoxophenylamino)-(4-trimethylsilanyloxyphenyl)-methyl]-5-trimethylsilanyloxypentanoyl}-4-phenyloxazolidin-2-on (10 mg) wird unter Argon und bei Eiskühlung in MTB-Ether (1 ml) suspendiert. N,O-Bistrimethylsilylacteamid (20,75 µl) wird zugegeben, gefolgt von Tetrabutylphosphoniumchlorid (4,2 mg) und Silber(I)oxid (3,2 mg). Es wird bei Raumtemperatur gerührt. Mittels Dünnschichtchromatographie und LC/MS-Vergleich wird eine Umsetzung zu 1-(4-Fluorophenyl)-3-[3-(4-fluorophenyl)-3-trimethylsilanyloxypropyl]-4-(4-trimethylsilanyloxy-phenyl)-azetidin-2-on festgestellt (M+H,-TMS,-HOTMS: 392).

### Beispiel 5

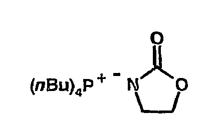

### Tetrabutylphosphoniumoxazolidia-2-on

Tetrabutylphosphoniumchlorid (300 mg) wird in Methanol (3 ml) gelöst und Silber(I)oxid (203,85 mg) zugegeben. Bei Raumtemperatur wird 16 Stunden lang unter Argon gerührt. Die Reaktionslösung wird über einen Spritzenfilter filtriert. Zum Filtrat wird Oxazolidin-2-on (104,4 mg) zugegeben und 2 Stunden lang bei Raumtemperatur gerührt. Das Lösungsmittel wird am Rotationsverdampfer entfernt. Tetrabutylphosphoniumoxazolidin-2-on (200 mg) wird als farbloses Öl erhalten. ¹H-NMR (d⁶-DMSO) 0.9 (m, 12 H), 1.4 (m, 16 H), 2.2 (m, 8 H), 3.4 (t, 2H), 3.85 (t, 2 H).

### Beispiel 6

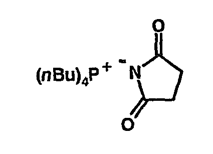

### Tetrabutylphosphoniumsuccinimid

Die Verbindung wird analog zur Vorschrift in Beispiel 5 aus Succinimid dargestellt. ¹H-NMR (d⁸-THF) 0.95 (m, 12 H), 1.4-1.6 (m, 16 H), 2.3 (s, 4 H), 2.55 (m, 8 H).

### Beispiel 7

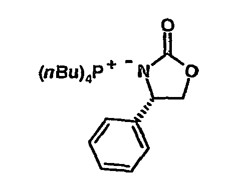

### Tetrabutylphosphonium-4-phenyloxazolidin-2-on

Die Verbindung wird analog zur Vorschrift in Beispiel 5 aus 4-Phenyloxazolidin-2-on dargestellt. ¹H-NMR (d⁶-DMSO) 0.9 (m, 12 H), 1.4 (m, 16 H), 2.2 (m, 8 H), 3.6 (t, 1H), 4.35 (t, 1 H), 4.75 (m, 1 H), 7.2-7.3 (m, 5 H).

### Beispiel 8

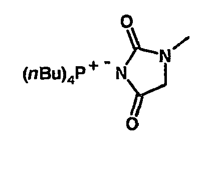

### Tetrabutylphosphonium-1-methyl-imidazolidin-2,4-dion

Die Verbindung wird analog zur Vorschrift in Beispiel 5 aus 1-Methyl-imidazolidin-2,4-dion dargestellt. ¹H-NMR (d⁶-DMSO) 0.9 (m, 12 H), 1.44 (m, 16 H), 2.15 (m, 8 H), 2.65 (s, 3H), 3.13 (s, 2 H).

### Beispiel 9

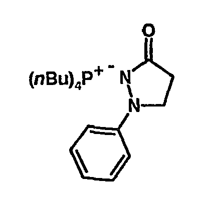

### Tetrabutylphosphonium-1-phenylpyrazolidin-3-on

Die Verbindung wird analog zur Vorschrift in Beispiel 5 aus 1-Phenylpyrazolidin-3-on dargestellt. ¹H-NMR (d⁶-DMSO) 0.9 (t, 12 H), 1.4 (m, 17 H), 1.55 (m, 1 H), 2.2 (m, 8 H), 5.5 (bs, 1 H), 6.95 (t, 1 H), 7.28 (t, 2 H), 7.53 (d, 2 H), 7.9 (s, 1 H).

### Beispiel 10

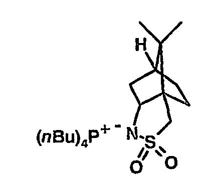

### Tetrabutylphosphonium-10,10-dimethyl-3-thia-4-aza-tricyclo[5.2.1.01,5]decan-3,3-dioxid

Die Verbindung wird analog zur Vorschrift in Beispiel 5 aus 2,10-Camphersultam dargestellt. ¹H-NMR (d⁶-DMSO) 0.75 (s, 3 H), 0.9 (t, 12 H), 1.0 (s, 3 H), 1.1-1.25 (m, 2 H), 1.4 (m, 16 H), 1.5-1.8 (m, 4 H), 2.2 (m, 8 H), 2.5 (m, 2 H), 3.05 (m, 1 H), 3.15 (d, 1 H).

### Beispiel 11

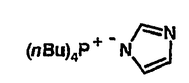

### Tetrabutylphosphoniumimidazolid

Die Verbindung wird analog zur Vorschrift in Beispiel 5 aus Imidazol dargestellt. ¹H-NMR (d⁶-DMSO) 0.9 (t, 12 H), 1.4 (m, 16 H), 2.2 (m, 8 H), 6.7 (s, 2 H), 7.15 (s, 1 H).

### Beispiel 12

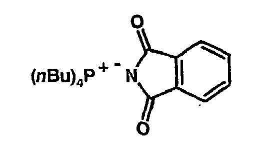

### Tetrabutylphosphoniumphthalimid

Die Verbindung wird analog zur Vorschrift in Beispiel 5 aus Phthalimid dargestellt. ¹H-NMR (d⁶-DMSO) 0.9 (t, 12 H), 1.4 (m, 16 H), 2.2 (m, 8 H), 7.38 (m, 2 H), 7.42 (m, 2 H).

### Beispiel 13

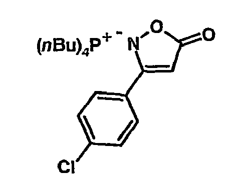

### Tetrabutylphosphonium-3-(4-chlorphenyl)-2H-isoxazol-5-on

Die Verbindung wird analog zur Vorschrift in Beispiel 5 aus 3-(4-Chlorphenyl)-2H-isoxazol-5-on dargestellt. ¹H-NMR (d⁶-DMSO) 0.9 (t, 12 H), 1.4 (m, 16 H), 2.2 (m, 8 H), 4.3 (bs, 1 H), 7.35 (m, 2 H), 7.55 (m, 2 H).

### Beispiel 14

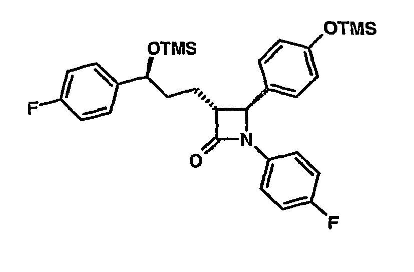

### Zyklisierung von 3-{5-(4-Fluorophenyl)-2-[(4-fluorophenylamino)-(4-trimethylsilanyl-oxyphenyl)-methyl]-5-trimethylsilanyloxypentanoyl}-4-phenyloxazolidin-2-on mit der Verbindung aus Beispiel 5

3-{5-(4-Fluorophenyl)-2-[(4-fluorophenylamino)-(4-trimethylsilanyloxyphenyl)-methyl]-5-trimethylsilanyloxypentanoyl}-4-phenyloxazolidin-2-on (10 mg) wird unter Argon und bei Eiskühlung in MTB-Ether (1 ml) suspendiert. N,O-Bistrimethylsilylacteamid (20,75 µl) wird zugegeben, gefolgt von einer katalytischen Menge Tetrabutylphosphoniumoxazolidin-2-on (3 mg) gelöst in MTB-Ether (100 µl). Es wird 1 Stunde lang bei Raumtemperatur gerührt. Mittels Dünnschichtchromatographie und LC/MS-Vergleich wird eine Umsetzung zu 1-(4-Fluorophenyl)-3-[3-(4-fluorophenyl)-3-trimethylsilanyloxy-propyl]-4-(4-trimethylsilanyloxy-phenyl)-azetidin-2-on festgestellt (M+H,-TMS,-HOTMS: 392).

### Beispiel 15

Die Zyklisierung von 3-{5-(4-Fluorophenyl)-2-[(4-Suorophenylamino)-(4-trimethylsilanyl-oxyphenyl)-methyl]-5-trimethylsilanyloxypentanoyl}-4-phenyloxazolidin-2-on zu 1-(4-Fluorophenyl)-3-[3-(4-fluorophenyl)-3-trimethylsilanyloxy-propyl]-4-(4-trimethylsilanyloxy-phenyl)-azetidin-2-on wird in analoger Weise zur Vorschrift in Beispiel 14, jedoch mit Tetrabutylphosphoniumsuccinimid aus Beispiel 6 als Zyklisierungskatalysator, durchgeführt. Die spektroskopischen Daten stimmen mit denen des Produkts aus Beispiel 10 überein (M+H,-TMS,-HOTMS: 392).

### Beispiel 16

Die Zyklisierung von 3-{5-(4-Fluorophenyl)-2-[(4-fluorophenylamino)-(4-trimethylsilanyl-oxyphenyl)-methyl]-5-trimethylsilanyloxypentanoyl}-4-phenyloxazolidin-2-on zu 1-(4-Fluorophenyl)-3-[3-(4-fluorophenyl)-3-trimethylsilanyloxy-propyl]-4-(4-trimethylsilanyloxy-phenyl)-azetidin-2-on wird in analoger Weise zur Vorschrift in Beispiel 14, jedoch mit Tetrabutylphosphonium-4-phenyloxazolidin-2-on aus Beispiel 7 als Zyklisierungskatalysator, durchgeführt. Die spektroskopischen Daten stimmen mit denen des Produkts aus Beispiel 10 überein (M+H,-TMS,-HOTMS: 392).

### Beispiel 17

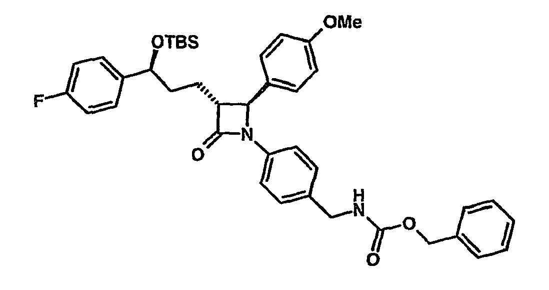

### Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester mit der Verbindung aus Beispiel 5

Unter Eiskühlung und in einer Argonatmosphäre wird {4-[5-(tert-Butyldimethylsilanyloxy)-5-(4-fluorophenyl)-1-(4-methoxy-phenyl)-2-(2-oxo-4-phenyloxazolidine-3-carbonyl)-pentyl-amino]-benzyl}-carbamylsäurebenzylester (500 mg) in MTB-Ether suspendiert (6 ml). N,O-Bistrimethylsilylacteamid (1,0 ml) wird zugegeben, gefolgt von Tetrabutylphosphoniumoxazolidin-2-on (40,8 mg) gelöst in MTB-Ether (1 ml). Es wird zwei Stunden lang bei Raumtemperatur gerührt. Danach wird eine 1N wäßrige Hydrogenchlorid-Lösung (HCl_{(aq)}) (1 ml), unter Eiskühlung Methanol (1 ml) und Tetrahydrofuran (2 ml) zugegeben. Über Nacht wird bei Raumtemperatur gerührt. Die Reaktionslösung wird eingeengt und mit Ethylacetat aufgenommen. Die organische Phase wird nacheinander mit 2N HCl_{(aq)}, gesättigter Natriumbicarbonatlösung und gesättigter Kochsalzlösung gewaschen. Danach wird über Natriumsulfat getrocknet, filtriert und das Lösungsmittel am Rotationsverdampfer entfernt. Nach Reinigung durch Chromatographie (1:6 Ethylacetat/n-Heptan) über eine SiO₂-Kartusche (5 g) wird {4-[3-[3-(tert-Butyldimethyl-silanyloxy)-3-(4-fluorophenyl)-propyl]-2-(4-methoxyphenyl)-4-oxo-azetidin-1-yl]-benzyl}-carbamylsäurebenzylester (240 mg) erhalten ¹H-NMR (d⁶-DMSO) -0.18 (s, 3 H), 0.02 (s, 3 H), 0.85 (s, 9 H), 1.75 (bs, 4 H), 3.05 (bs, 1 H), 3.7 (s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10 H), 7.73 (t, 1 H).

### Beispiel 18

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester zu {4-[3-[3-(tert-Butyldimethyl-silanyloxy)-3-(4-fluorophenyl)-propyl]-2-(4-methoxyphenyl)-4-oxo-azetidin-1-yl]-benzyl}-carbamylsäurebenzylester wird in analoger Weise zur Vorschrift in Beispiel 17, jedoch mit Tetrabutylphosphonium-1-methyl-imidazolidin-2,4-dion aus Beispiel 8 als Zyklisierungskatalysator, durchgeführt. Die spektroskopischen Daten stimmen mit denen des Produkts aus Beispiel 17 überein: ¹H-NMR (d⁶-DMSO) -0.18 (s, 3 H), 0.02 (s, 3 H), 0.85 (s, 9 H), 1.75 (bs, 4 H), 3.05 (bs, 1 H), 3.7 (s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10 H), 7.73 (t, 1 H).

### Beispiel 19

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester zu {4-[3-[3-(tert-Butyldimethyl-silanyloxy)-3-(4-fluorophenyl)-propyl]-2-(4-methoxyphenyl)-4-oxo-azetidin-1-yl]-benzyl}-carbamylsäurebenzylester wird in analoger Weise zur Vorschrift in Beispiel 17, jedoch mit Tetrabutylphosphonium-1-phenylpyrazolidin-3-on aus Beispiel 9 als Zyklisierungskatalysator, durchgeführt. Die spektroskopischen Daten stimmen mit denen des Produkts aus Beispiel 17 überein: ¹H-NMR (d⁶-DMSO) -0.18 (s, 3 H), 0.02 (s, 3 H), 0.85 (s, 9 H), 1.75 (bs, 4 H), 3.05 (bs, 1 H), 3.7 (s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10 H), 7.73 (t, 1 H).

### Beispiel 20

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester zu {4-[3-[3-(tert-Butyldimethyl-silanyloxy)-3-(4-fluorophenyl)-propyl]-2-(4-methoxyphenyl)-4-oxo-azetidin-1-yl]-benzyl}-carbamylsäurebenzylester wrd in analoger Weise zur Vorschrift in Beispiel 17, jedoch mit Tetrabutylphosphonium-10,10-dimethyl-3-thia-4-aza-tricyclo[5.2.1.01,5]decan-3,3-dioxid aus Beispiel 10 als Zyklisierungskatalysator, durchgeführt. Die spektroskopischen Daten stimmen mit denen des Produkts aus Beispiel 17 überein: ¹H-NMR (d⁶-DMSO) -0.18 (s, 3 H), 0.02 (s, 3 H), 0.85 (s, 9 H), 1.75 (bs, 4 H), 3.05 (bs, 1 H), 3.7(s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10H), 7.73 (t, 1 H).

### Beispiel 21

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester zu {4-[3-[3-(tert-Butyldimethyl-silanyloxy)-3-(4-fluorophenyl)-propyl]-2-(4-methoxyphenyl)-4-oxo-azetidin-1-yl]-benzyl}-carbamylsäurebenzylester wird in analoger Weise zur Vorschrift in Beispiel 17, jedoch mit Tetrabutylphosphoniumimidazolid aus Beispiel 11 als Zyklisierungskatalysator, durchgeführt. Die spektroskopischen Daten stimmen mit denen des Produkts aus Beispiel 17 überein: ¹H-NMR (d⁶-DMSO) -0.18 (s, 3 H), 0.02 (s, 3 H), 0.85 (s, 9 H), 1.75 (bs, 4 H), 3.05 (bs, 1 H), 3.7 (s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10 H), 7.73 (t, 1 H).

### Beispiel 22

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester zu {4-[3-[3-(tert-Butyldimethyl-silanyloxy)-3-(4-fluorophenyl)-propyl]-2-(4-methoxyphenyl)-4-oxo-azetidin-1-yl]-benzyl}-carbamylsäurebenzylester wird in analoger Weise zur Vorschrift in Beispiel 17, jedoch mit Tetrabutylphosphoniumphthalimid aus Beispiel 12 als Zyklisierungskatalysator, durchgeführt. Die spektroskopischen Daten stimmen mit denen des Produkts aus Beispiel 17 überein: ¹H-NMR (d⁶-DMSO) -0.18 (s, 3 H), 0.02 (s, 3 H), 0.85 (s, 9 H), 1.75 (bs, 4 H), 3.05 (bs, 1H), 3.7 (s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10 H), 7.73 (t, 1 H).

### Beispiel 23

Die Zyklisierung von {4-[5-(tert-Butyldimethylsilanyloxy)-5-(4-ffuorophenyl)-1-(4-methoxy-phenyl)-2-(2-oxo-4-phenyloxazolidine-3-carbonyl)-pentylamino]-benzyl}-carbamylsäure-benzylester zu {4-[3-[3-(tert- Butyldimethyl-silanyloxy)-3-(4-fluorophenyl)-propyl]-2-(4-methoxyphenyl)-4-oxo-azetidin-1-yl]-benzyl}-carbamylsäurebenzylester wird in analoger Weise zur Vorschrift in Beispiel 17, jedoch mit Tetrabutylphosphonium-3-(4-chlorphenyl)-2H-isoxazol-5-on aus Beispiel 13 als Zyklisierungskatalysator, durchgeführt. Die spektroskopischen Daten stimmen mit denen des Produkts aus Beispiel 17 überein: ¹H-NMR (d⁶-DMSO) -0.18 (s, 3 H), 0.02 (s, 3 H), 0.85 (s, 9 H), 1.75 (bs, 4 H), 3.05 (bs, 1 H), 3.7 (s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10 H), 7.73 (t, 1 H).

### Beispiel 24

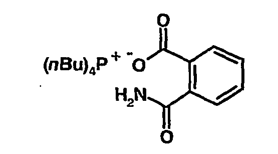

### Tetrabutylphosphoniumphthalamate

Tetrabutylphosphoniumphthalamat wird auch auf folgendem Weg dargestellt. Eine Lösung von Tetrabutylphosphoniumhydroxid (12,5 g; 40% in Wasser) wird vorgelegt. Dann wird Phthalimid (2,7 g) zugegeben und die Reaktionslösung wird über Nacht bei Raumtemperatur gerührt. Danach wird das Wasser am Rotationsverdampfer entfernt und der Rückstand in Toluol aufgenommen. Das Toluol wird am Rotationsverdampfer entfernt und der Rückstand wird wieder in Toluol aufgenommen. Diese Prozedur wird 4-5 mal wiederholt. Danach wird das Lösungsmittel bis zur Trockene entfernt. ¹H-NMR (d⁶-DMSO) 0.9 (t, 12 H), 1.42 (m, 16 H), 2.18 (m, 8 H), 7.05 (b, 1 H), 7.18 (m, 1 H), 7.29 (d, 2H), 7.74 (d, 1H), 10.35 (b, 1H).

### Beispiel 25

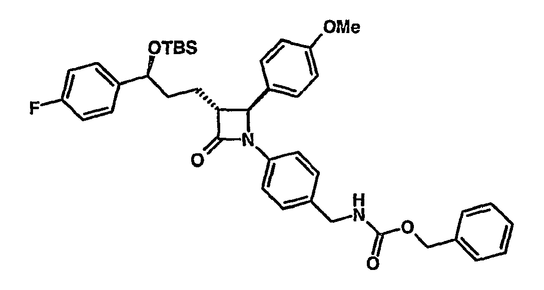

### Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylmino]-benzyl}-carbamylsäure-benzylester mit Tetrabutylphosphoniumphthalimid aus Beispiel 24

Unter Eiskühlung und in einer Argonatmosphäre wird {4-[5-(tert-Butyldimethylsilanyloxy)-5-(4-fluorophenyl)-1-(4-methoxy-phenyl)-2-(2-oxo-4-phenyloxazolidine-3-carbonyl)-pentyl-amino]-benzyl}-carbamylsäurebenzylester (100 mg) in MTB-Ether suspendiert (5 ml). N,O-Bistrimethylsilylacteamid (0,17 ml) wird zugegeben, gefolgt von Tetrabutylphosphoniumphthalamat (12 mg) gelöst in MTB-Ether (1 ml). Es wird 2-3 Stunden lang bei 50° C gerührt. Die Reaktionslösung wird eingeengt und mit Ethylacetat aufgenommen. Die organische Phase wird nacheinander mit 2N HCl_{(aq)}, gesättigter Natriumbicarbonatlösung und gesättigter Kochsalzlösung gewaschen. Danach wird über Natriumsulfat getrocknet, filtriert und das Lösungsmittel am Rotationsverdampfer entfernt. Nach Reinigung durch Chromatographie (Jones Chromatographie Flashmaster) wird {4-[3-[3-(tert-Butyldimethyl-silanyloxy)-3-(4-fluorophenyl)-propyl]-2-(4-methoxyphenyl)-4-oxo-azetidin-1-yl]-benzyl}-carbamylsäurebenzylester (41 mg) erhalten ¹H-NMR (d⁶-DMSO) -0.18 (s, 3 H), 0.02 (s, 3 H), 0.85 (s, 9 H), 1.75 (bs, 4 H), 3.05 (bs, 1 H), 3.7 (s, 3 H), 4.1 (d, 2 H), 4.72 (m, 1 H), 4.82 (s, 1 H), 5.0 (s, 2H), 6.9 (d, 2 H), 7.12 (m, 5 H), 7.35 (m, 10 H), 7.73 (t, 1 H).

### Beispiel 26

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester mit Tetrabutylphosphoniumphthalamat aus Beispiel 24 wird in analoger Weise zur Vorschrift in Beispiel 25, jedoch in Diisopropylether und bei 50°C innerhalb von 6 Stunden durchgeführt. Mittels LC/MS-Vergleich (Liquid-Chromatographyy/Mass-Spectrometry) wird eine Umsetzung zum analogen Produkt aus Beispiel 25 festgestellt.

### Beispiel 27

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester mit Tetrabutylphosphoniumphthalamat aus Beispiel 24 wird in analoger Weise zur Vorschrift in Beispiel 25, jedoch in Toluol und bei 60°C innerhalb von 13 Stunden durchgeführt. Mittels LC/MS-Vergleich (Liquid-Chromatography/Mass-Spectrometry) wird eine Umsetzung zum analogen Produkt aus Beispiel 25 festgestellt.

### Beispiel 28

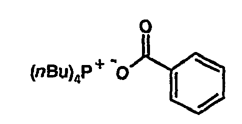

### Tetrabutylphosphoniumbenzoat

Tetrabutylphosphoniumbenzoat wird auch auf folgendem Weg dargestellt. Eine Lösung von Tetrabutylphosphoniumhydroxid (4,7 g; 40% in Wasser) wird vorgelegt. Dann wird Benzoesäure (0,8 g) zugegeben und die Reaktionslösung wird für 5 Stunden bei Raumtemperatur gerührt. Danach wird das Wasser am Rotationsverdampfer entfernt und der Rückstand in Toluol aufgenommen. Das Toluol wird am Rotationsverdampfer entfernt und der Rückstand wird wieder in Toluol aufgenommen. Diese Prozedur wird 4-5 mal wiederholt. Danach wird das Lösungsmittel bis zur Trockene entfernt.

### Beispiel 29

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester mit Tetrabutylphosphoniumbenzoat aus Beispiel 28 wird in analoger Weise zur Vorschrift in Beispiel 25, jedoch in Diisopropylether und bei 60°C innerhalb von 3 Stunden durchgeführt. Mittels LC/MS-Vergleich (Liquid-Chromatographyy/Mass-Spectrometry) wird eine Umsetzung zum analogen Produkt aus Beispiel 25 festgestellt.

### Beispiel 30

Die Zyklisierung von {4-[5-(tert-Butyldimethylsilanyloxy)-5-(4-fluorophenyl)-1-(4-methoxy-phenyl)-2-(2-oxo-4-phenyloxazolidine-3-carbonyl)-pentylamino]-benzyl}-carbamylsäure-benzylester mit Tetrabutylphosphoniumacetat (kommerzielle Ware wurde im Vakuum getrocknet) wird in analoger Weise zur Vorschrift in Beispiel 25, jedoch in Diisopropylether und bei 60°C innerhalb von 0,5 Stunden durchgeführt. Mittels LC/MS-Vergleich (Liquid-Chromatographyy/Mass-Spectrometry) wird eine Umsetzung zum analogen Produkt aus Beispiel 25 festgestellt.

## Patentansprüche

1. Verfahren zur Herstellung von 1,4-Diphenylazetidinon-Derivaten aus in geeigneter Weise geschützten β-substituierten Aminoamiden in Gegenwart von Silylierungsmitteln und mindestens einem Zyklisierungskatalysator, wobei dieser Zyklisierungskatalysator durch eine der nachstehenden allgemeinen Formeln 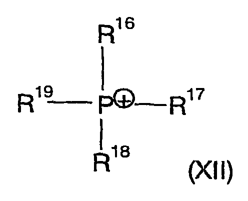 als Kation, wobei R¹⁶, R¹⁷, R¹⁸, R¹⁹ unabhängig voneinander Aryl, (C₁-C₁₅)Alkyl, Benzyl bedeuten,
R⁴¹ Aryl, (C₁-C₁₅)Alkyl, Benzyl bedeutet,
R⁴² (C₁-C₁₅)Alkyl, Benzyl, (C₅-C₈)Cycloalkyl, Aryl, wobei Aryl substituiert sein kann mit F, Cl, Br, J, -OH, -O(C₁-C₃)Alkyl, -NH₂, -NH(C₁-C₃)Alkyl, -N[(C₁-C₃)Alkyl]₂, C(O)OH, -C(O)O(C₁-C₃)Alkyl, -C(O)NH₂, -C(O)NH(C₁-C₃)Alkyl, -C(O)N[(C₁-C₃)Alkyl]₂, -SO₂NH₂, -SO₂NH(C₁-C₃)Alkyl, -SO₂N[(C₁-C₃)Alkyl]₂, -CN, (C₁-C₁₂)Alkyl und (C₅-C₈)Cycloalkyl, bedeutet,
und 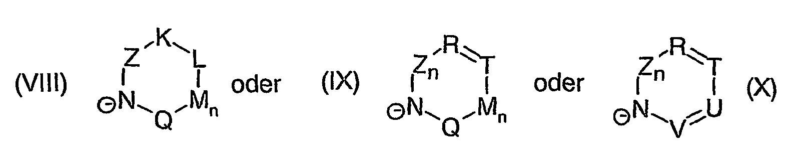 oder 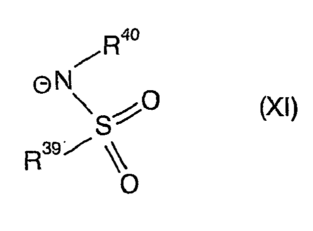 als Anion dargestellt wird
und die Symbole, Substituenten und Indices folgende Bedeutung haben,
Z = C=O, C=S, S=O, SO₂ oder C=NR²⁰
K = O, S, NR²¹ oder CR²²R²³
L = NR²⁴ oder CR²⁵R²⁶
n = 0 oder 1
M = O, C=O, NR²⁷ oder CR²⁸R²⁹
Q = O, S, NR³⁰, CR³¹R³², C=O, C=S, S=O, SO₂ oder C=NR³⁴
R = CR³⁵ oder N
T = CR³⁶ oder N
U = CR³⁷ oder N
V = CR³⁸ oder N
wobei R²⁰ bis R³² und R³⁴ bis R³⁸ unabhängig voneinander H, (C₁-C₆)Alkyl, Aryl oder Heteroaryl bedeuten und jeweils zwei Alkylreste gemeinsam auch einen Cycloalkylenrest von maximal 6 C-Bausteinen im Ring bilden können, der wiederum substituiert sein kann mit F, Cl, Br, I, CF₃, NO₂, COO(C₁-C₆)Alkyl, CON[(C₁-C₆)Alkyl]₂, Cycloalkyl, (C₁-C₁₀)-Alkyl, (C₂-C₆)Alkenyl, O-(C₁-C₆)Alkyl, O-CO-(C₁-C₆)Alkyl, O-CO-(C₁-C₆)Alkylen-aryl, SO₂N[(C₁-C₆)Alkyl]₂, S-(C₁-C₆)Alkyl, S-(CH₂-)ₙAryl, SO-(C₁-C₆)Alkyl, SO-(CH₂-)ₙAryl, SO₂-(C₁-C₆)Alkyl, SO₂-(CH₂-)ₙAryl, SO₂-N((C₁-C₆)-Alkyl)(CH₂-)ₙAryl, oder SO₂-N((CH₂-)ₙAryl)₂, wobei n = 0 bis 6 sein kann und der Arylrest bis zu zweifach mit F, Cl, Br, CF₃, SF₅, NO₂, OCF₃, O-(C₁-C₆)Alkyl oder (C₁-C₆)Alkyl substituiert sein kann; oder mit N((C₁-C₆)Alkyl)₂, NH-CO-NH-(C₁-C₆)Alkyl], NH-CO-NH-Aryl, N[(C₁-C₆)Alkyl]-CO-(C₁-C₆)Alkyl, N[(C₁-C₆)Alkyl]-COO-(C₁-C₆)Alkyl, N[(C₁-C₆)Alkyl]-CO-Aryl, N[(C₁-C₆)Alkyl]-COO-Aryl, N[(C₁-C₆)Alkyl]-CO-N((C₁-C₆)Alkyl)₂, N[(C₁-C₆)Alkyl]-CO-N((C₁C₆)Alkyl)-Aryl, N[(C₁-C₆)Alkyl]-CO-N(Aryl)₂, N(Aryl)-CO-(C₁-C₆)Alkyl, N(Aryl)-COO-(C₁-C₆)Alkyl, N(Aryl)-CO-Aryl, N(Aryl)-COO-Aryl, N(Aryl)-CO-N((C₁-C₆)Alkyl)₂, N(Aryl)-CO-N[(C₁-C₆)Alkyl]-Aryl, N(Aryl)-CO-N(Aryl)₂, Aryl, O-(CH₂-)ₙAryl, wobei n = 0 bis 6 sein kann, wobei der Aryl-Rest 1 bis 3-fach substituiert sein kann mit F, Cl, Br, I, CF₃, NO₂, OCF₃, O-(C₁-C₆)Alkyl, (C₁-C₆)Alkyl, N((C₁-C₆)Alkyl)₂, SF₅, SO₂-CH₃ oder COO-(C₁-C₆)Alkyl
und wobei R³⁹ und R⁴⁰ unabhängig voneinander (C₁-C₆)Alkyl, wobei ein oder mehrere nicht benachbarte C-Atome durch NH oder C=O ersetzt sein können, (C₁-C₆)-Perfluoralkyl, Aryl oder Heteroaryl bedeuten, oder R³⁹ und R⁴⁰ bilden zusammen ein 1,8-Naphthyl oder 1,7,7-Trimethyl-bicyclo[2.2.1]heptanyl, R⁴⁰ kann auch H sein,
oder wobei in diesem Zyklisierungskatalysator das Kation dem der allgemeinen Formel (XII) entspricht und das Anion R⁴¹O⁻ ist, oder wobei in diesem Zyklisierungskatalysator das Kation dem der allgemeinen Formel (XII) entspricht und das Anion R⁴²COO⁻ ist, oder wobei in diesem Zyklisierungskatalysator das Kation der allgemeinen Formel (XII) entspricht, und das Anion Cl⁻, Br⁻ oder I⁻ ist und diese mit Ag₂O kombiniert werden.

2. Verfahren zur Herstellung von 1,4-Diphenylazetidinon-Derivaten nach Anspruch 1, wobei diese durch die allgemeine Formel (I) dargestellt sind, 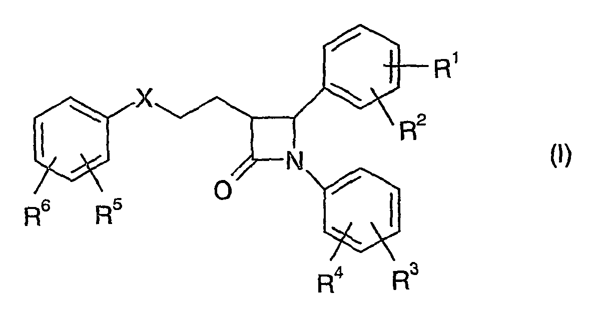 in der die Symbole, Substituenten und Indices folgende Bedeutung haben,
X = CH₂, CHOH, CO oder CHOCOR¹¹
R¹, R² = unabhängig voneinander H, OH, OCF₃, oder O-(C₁-C₆)Alkyl, O-(C₃-C₇)Cycloalkyl, O-COR¹¹, CN, CH₂NHR⁷, CH₂NR⁷R⁸, NR⁷R⁸, COR¹⁴, F oder Cl
R³, R⁴= unabhängig voneinander H, F, Cl, OH, OCF₃, O-(C₁-C₆)Alkyl, O-(C₃-C₇)Cycloalkyl, O-COR¹¹, CN, CH₂NHR⁷, CH₂NR⁷R⁸, NR⁷R⁸, COR¹⁴ oder (C₁-C₆)Akyl
R⁵, R⁶ = unabhängig voneinander H, F, Cl, (C₁-C₆)Alkyl, CF₃ oder OCF₃
R⁷ = H, C(=O)-Y(-CH₂)ₖ-Y-C(=O)R⁹ oder C(=O)-Y(-CH₂)ₖ-NHR¹⁰
k = 2 bis 16
Y = Einfachbindung oder NR¹³
R⁸ = H, (C₁-C₆)Alkyl, oder (C₃-C₇)Cycloalkyl
R⁹ = OH oder NHCH₂[-CH(OH)]ₘ-CH₂OH oder eine in geeigneter Weise geschütze Form davon
R¹⁰ = H, C(=O)[-CH(OH)]ₘ-CH₂OH oder eine in geeigneter Weise geschützte Form davon
m = 0 bis 5
R¹¹ = H, (C₁-C₆)Alkyl, (C₃-C₇)Cycloalkyl, (substituiertes)Phenyl oder OR¹²
R¹² = (C₁-C₆)Alkyl oder (C₃-C₇)Cycloalkyl
R¹³ = (C₁-C₆)Alkyl oder (C₃-C₇)Cycloalkyl, Aryl oder Heteroaryl
R¹⁴ = OH, OR¹², NR¹³(-CH₂)ₙ-Y-C(=O)R⁹ oder NR¹³(-CH₂)ₙ-NHR¹⁰
in Gegenwart eines Silylierungsmittels und eines Zyklisierungskatalysators, **dadurch gekennzeichnet, dass** Verbindungen der allgemeinen Formel (IV) 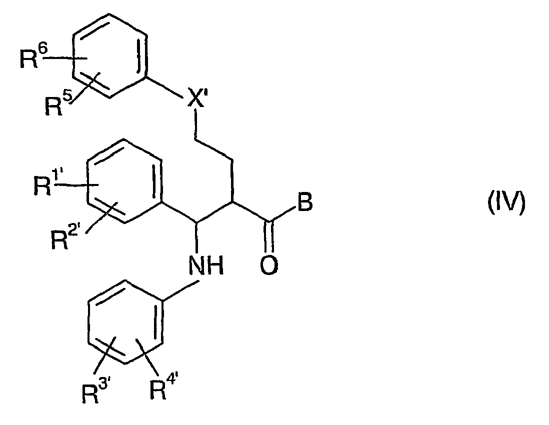 in der die Symbole, Substituenten und Indices - soweit nicht vorstehend definiert - folgende Bedeutung haben,
X' = X, CHOSi(Alkyl)ₒ(Aryl)ₚ mit o, p = 1 bis 3 und o + p = 3, C(OAlkyl)₂ oder zyklisches Ketal wie C[O(-CH₂)_{q}-O] mit q = 2, 3
R^{1'}, R^{2'} = R¹, R² und O-Schutzgruppe
R^{3'}, R^{4'} = R³, R⁴, CH₂NHCO₂CH₂(C₆H₅), CH₂N[Si(Alkyl)ₒ(Aryl)ₚ]CO₂CH₂(C₆H₅), CH₂NHCO₂-tert.Bu, CH₂N[Si(Alkyl)ₒ(Phenyl)ₚ]CO₂-tert.Bu, CH₂NHC(C₆H₅)₃, CH₂N=C(C₆H₅)₂ oder CH₂N=CH[C₆H₄(R⁶)] 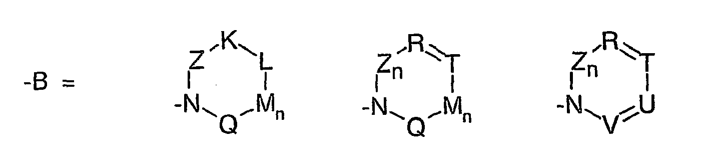
oder 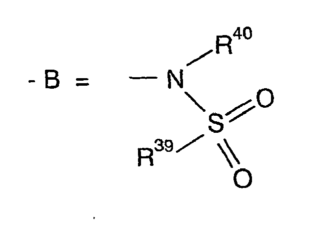 zu (Vor-)Produkten der allgemeinen Formel (V) zyklisiert werden, die zur Verbindung (I) entschützt werden können, 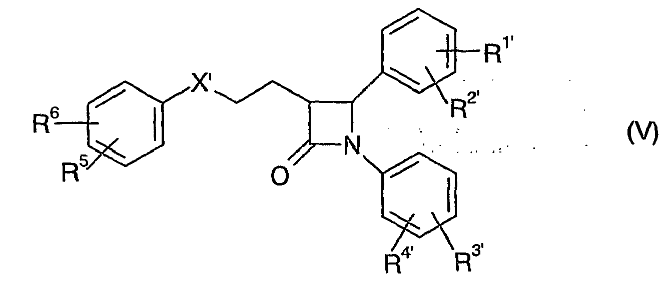 wobei der Zyklisierungskatalysator durch eine der nachstehenden allgemeinen Formeln 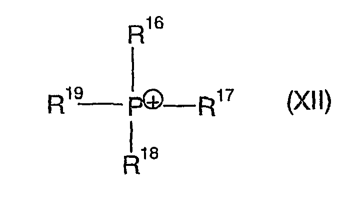 als Kation, wobei R¹⁶, R¹⁷, R¹⁸, R¹⁹ unabhängig voneinander Aryl, (C₁-C₁₅)Alkyl, Benzyl bedeuten,
R⁴¹ Aryl, (C₁-C₁₅)Alkyl, Benzyl bedeutet,
R⁴² (C₁-C₁₅)Alkyl, Benzyl, (C₅-C₈)Cycloalkyl, Aryl, wobei Aryl substituiert sein kann mit F, Cl, Br, J, -OH, -O(C₁-C₃)Alkyl, -NH₂, -NH(C₁-C₃)Alkyl, -N[(C₁-C₃)Alkyl]₂, -C(O)OH, -C(O)O(C₁-C₃)Alkyl, -C(O)NH₂, -C(O)NH(C₁-C₃)Alkyl,-C(O)N[(C₁-C₃)Alkyl]₂, -SO₂NH₂, SO₂NH(C₁-C₃)Alkyl, -SO₂N[(Ci-C3)Alkyl]₂,-CN, (C₁-C₁₂)Alkyl und (C₅-C₈)Cycloalkyl, bedeutet,
und 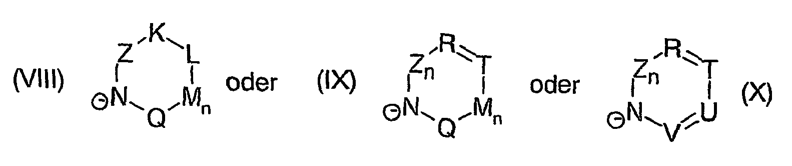 oder 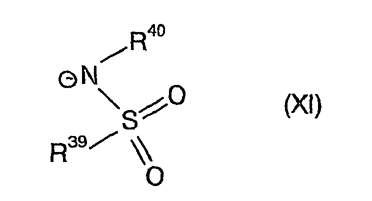 als Anion dargestellt wird,
oder wobei in diesem Zyklisierungskatalysator das Kation dem der allgemeinen Formel (XII) entspricht und das Anion R⁴¹O⁻ ist, oder wobei in diesem Zyklisierungskatalysator das Kation dem der allgemeinen Formel (XII) entspricht und das Anion R⁴²COO⁻ ist, oder wobei in diesem Zyklisierungskatalysator das Kation der allgemeinen Formel (XII) entspricht und das Anion Cl⁻, Br⁻ oder I⁻ ist und diese mit Ag₂O kombiniert werden.

3. Verfahren nach Anspruch 2, **dadurch gekennzeichnet, dass** Verbindungen der allgemeinen Formel (II) 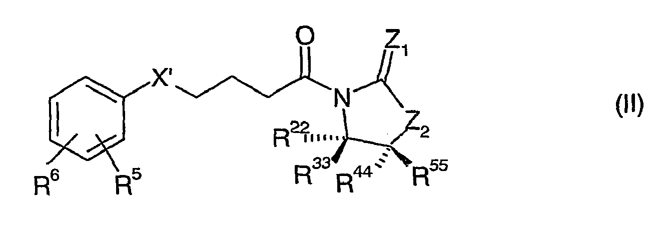 mit Iminen der allgemeinen Formel (III) 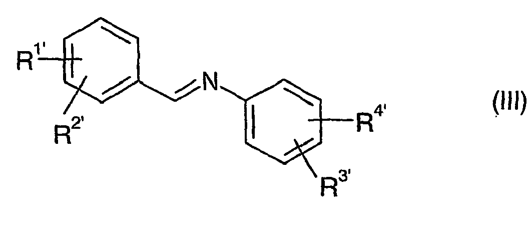 zu Zwischenstufen der allgemeinen Formel (IV'), 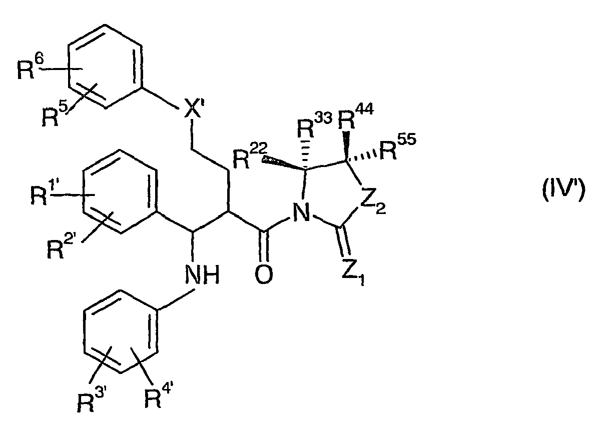 umgesetzt werden, die dann zu (Vor)Produkten der allgemeinen Formel (V) zyklisiert werden, die zur Verbindung (I) entschützt werden können, 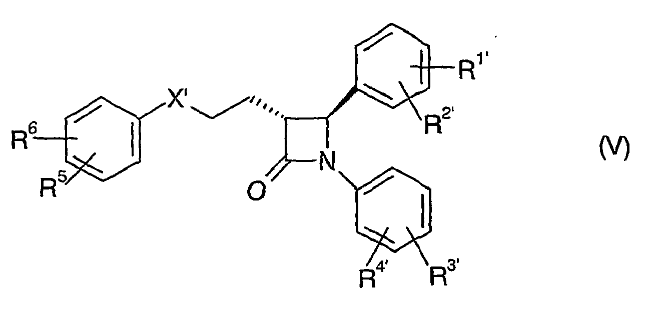 in der die Symbole, Substituenten und Indices - soweit nicht vorstehend definiert - folgende Bedeutung haben,
X' = X, CHOSi(Alkyl)ₒ(Aryl)p mit o, p = 1 bis 3 und o + p = 3, C(OAlkyl)₂ oder zyklisches Ketal wie C[O(-CH₂)q-O] mit q = 2, 3
R²², R³³, R⁴⁴, R⁵⁵ = unabhängig voneinander H, Aryl oder (C₁-C₁₀)Alkyl
Z_{1,} Z₂ = unabhängig voneinander O, NH, NR¹⁵, oder S
R¹⁵ = Aryl oder (C₁-C₁₀)Alkyl,
R^{1'}, R^{2'} = R¹, R² und O-Schutzgruppe
R^{3'}, R^{4'}= R³, R⁴, CH₂NHCO₂CH₂(C₆H₅), CH₂N[Si(Alkyl)ₒ(Aryl)ₚ]CO₂CH₂(C₆H₅), CH₂NHCO₂-tert.Bu, CH₂N[Si(Alkyl)ₒ(Phenyl)ₚ]CO₂-tert.Bu, CH₂NHC(C₆H₅)₃, CH₂N=C(C₆H₅)₂ oder CH₂N=CH[C₆H₄(R⁶)]
wobei der Zyklisierungskatalysator durch eine der allgemeinen Formeln (VIa) bis (VII) dargestellt wird 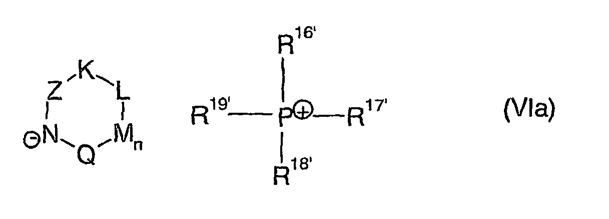 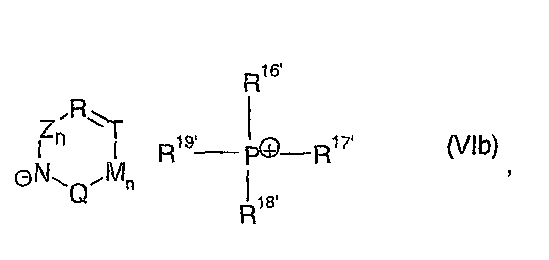 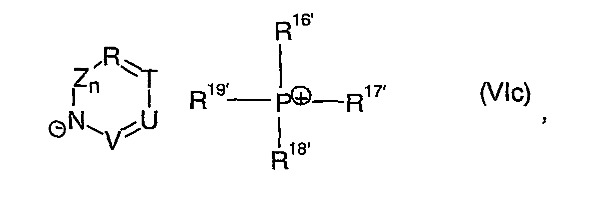 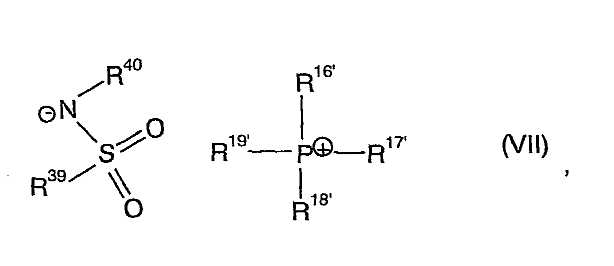 wobei R^{16'}, R^{17'}, R^{18'}, R^{19'} unabhängig voneinander Aryl, (C₁-C₁₅)Alkyl, Benzyl bedeuten.

4. Verfahren nach Anspruch 2, **dadurch gekennzeichnet, dass** Verbindungen der allgemeinen Formel (II) 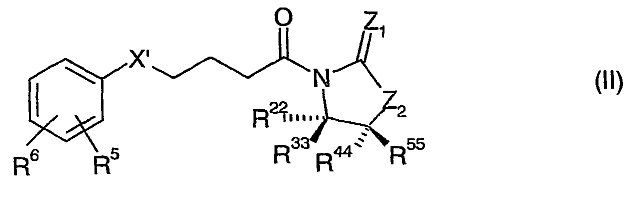 mit Iminen der allgemeinen Formel (III) 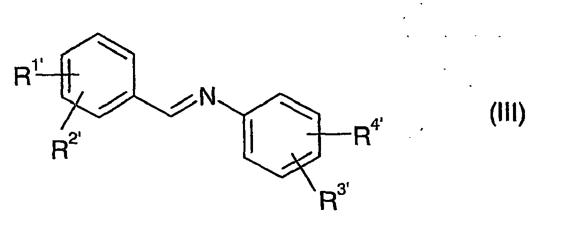 zu Zwischenstufen der allgemeinen Formel (IV'), 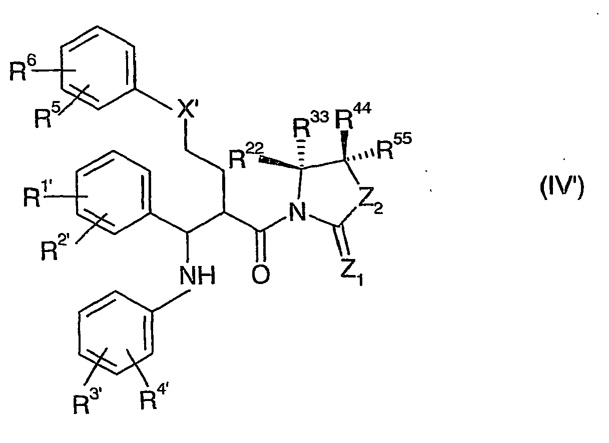 umgesetzt werden, die dann zu (Vor)Produkten der allgemeinen Formel (V) zyklisiert werden, die zur Verbindung (I) entschützt werden können, 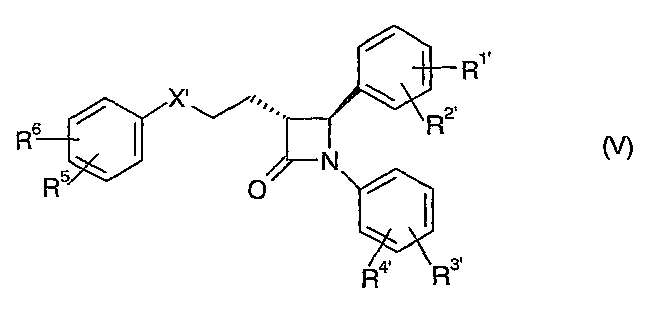 in der die Symbole, Substituenten und Indices - soweit nicht vorstehend definiert - folgende Bedeutung haben,
X' = X, CHOSi(Alkyl)ₒ(Aryl)ₚ mit o, p = 1 bis 3 und o + p = 3, C(OAlkyl)₂ oder zyklisches Ketal wie C[O(-CH₂)_{q}-O] mit q = 2, 3
R²², R³³, R⁴⁴, R⁵⁵ = unabhängig voneinander H, Aryl oder (C₁-C₁₀)Alkyl
Z₁, Z₂ = unabhängig voneinander O, NH, NR¹⁵, oder S
R¹⁵ = Aryl oder (C₁-C₁₀)Alkyl,
R^{1'}, R^{2'} = R¹, R² und O-Schutzgruppe
R^{3'}, R^{4'} = R³, R⁴, CH₂NHCO₂CH₂(C₆H₅), CH₂N[Si(Alkyl)ₒ(Aryl)ₚ]CO₂CH₂(C₆H₅), CH₂NHCO₂-tert.Bu, CH₂N[Si(Alkyl)ₒ(Phenyl)ₚ]CO₂-tert.Bu, CH₂NHC(C₆H₅)₃, CH₂N=C(C₆H₅)₂ oder CH₂N=CH[C₆H₄(R⁶)]
wobei im Zyklisierungskatalysator das Kation dem der allgemeinen Formel (XII) entspricht und das Anion R⁴¹O⁻ ist, oder wobei in diesem Zyklisierungskatalysator das Kation dem der allgemeinen Formel (XII) entspricht und das Anion R⁴²COO⁻ ist, oder wobei in diesem Zyklisierungskatalysator das Kation der allgemeinen Formel (XII) entspricht und das Anion Cl⁻, Br⁻ oder I⁻ ist und diese mit Ag₂O kombiniert werden, als Kation, wobei
R¹⁶, R¹⁷, R¹⁸, R¹⁹ unabhängig voneinander Aryl, (C₁-C₁₅)Alkyl, Benzyl bedeuten,
R⁴¹ Aryl, (C₁-C₁₅)Alkyl, Benzyl bedeutet,
R⁴² (C₁-C₁₅)Alkyl, Benzyl, (C₅-C₈)Cycloalkyl, Aryl, wobei Aryl substituiert sein kann mit F, Cl, Br, J, -OH, -O(C₁-C₃)Alkyl, -NH₂, -NH(C₁-C₃)Alkyl, -N[(C₁-C₃)Alkyl]₂, -C(O)OH, -C(O)O(C₁-C₃)Alkyl, -C(O)NH₂, -C(O)NH(C₁-C₃)Alkyl, - C(O)N[(C₁-C₃)Alkyl]₂, -SO₂NH₂, -SO₂NH(C₁-C₃)Alkyl, -SO₂N[(C₁-C₃)Alkyl]₂, - CN, (C₁-C₁₂)Alkyl und (C₅-C₈)Cycloalkyl, bedeutet.

5. Verfahren nach Anspruch 2, **dadurch gekennzeichnet, dass** Verbindungen der allgemeinen Formel (II) 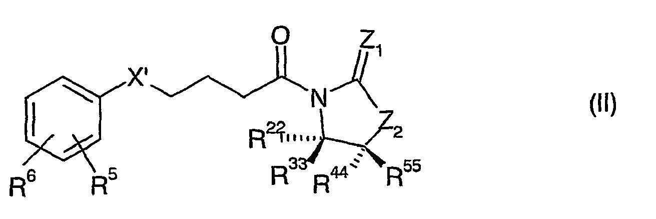 mit Iminen der allgemeinen Formel (III) 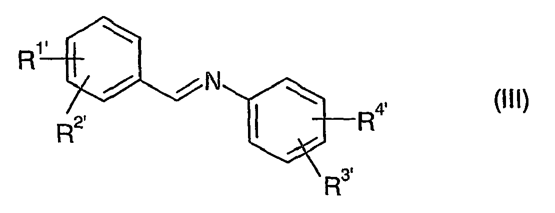 zu Zwischenstufen der allgemeinen Formel (IV'), 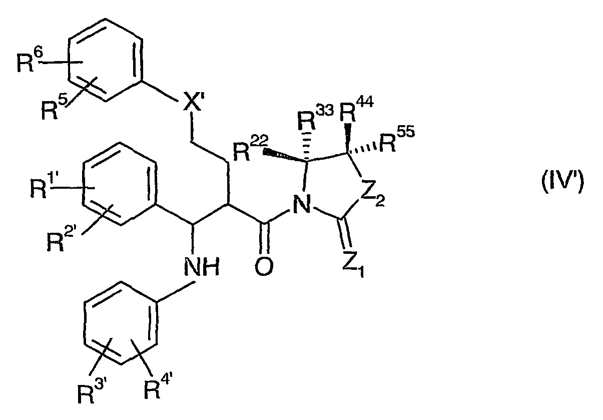 umgesetzt werden, die dann zu (Vor)Produkten der allgemeinen Formel (V) zyklisiert werden, die zur Verbindung (I) entschützt werden können, 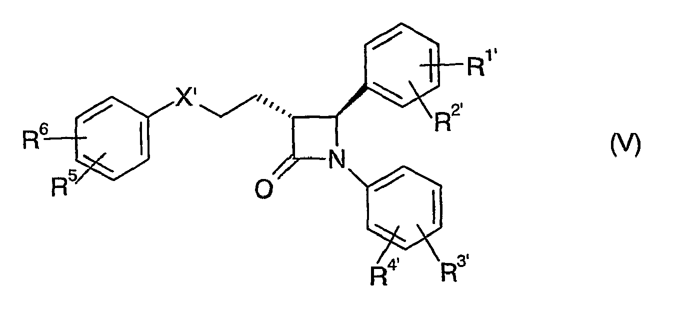 in der die Symbole, Substituenten und Indices - soweit nicht vorstehend definiert - folgende Bedeutung haben,
X' = X, CHOSi(Alkyl)ₒ(Aryl)ₚ mit o, p = 1 bis 3 und o + p = 3, C(OAlkyl)₂ oder zyklisches Ketal wie C[O(-CH₂)_{q}-O] mit q = 2, 3
R²², R³³, R⁴⁴, R⁵⁵ = unabhängig voneinander H, Aryl oder (C₁-C₁₀)Alkyl
Z₁, Z₂ = unabhängig voneinander O, NH, NR¹⁵, oder S
R¹⁵ = Aryl oder (C₁-C₁₀)Alkyl,
R^{1'}, R^{2'} = R¹, R² und O-Schutzgruppe
R^{3'}, R^{4'} = R³, R⁴, CH₂NHCO₂CH₂(C₆H₅), CH₂N[Si(Alkyl)ₒ(Aryl)ₚ]CO₂CH₂(C₆H₅), CH₂NHCO₂-tert.Bu, CH₂N[Si(Alkyl)ₒ(Phenyl)ₚ]CO₂-tert.Bu, CH₂NHC(C₆H₅)₃, CH₂N=C(C₆H₅)₂ oder CH₂N=CH[C₆H₄(R⁶)]
wobei im Zyklisierungskatalysator das Kation dem der allgemeinen Formel (XII) entspricht und das Anion R⁴²COO⁻ ist, wobei
R¹⁶, R¹⁷, R¹⁸, R¹⁹ unabhängig voneinander Aryl, (C₁-C₁₀)Alkyl, Benzyl bedeuten,
R⁴¹ Aryl, (C₁-C₁₀)Alkyl, Benzyl bedeutet,
R⁴² (C₁-C₁₅)Alkyl, Benzyl, (C₅-C₈)Cycloalkyl, Aryl, wobei Aryl substituiert sein kann mit F, Cl, Br, J, -OH, -O(C₁-C₃)Alkyl, -NH₂, -NH(C₁-C₃)Alkyl, -N[(C₁-C₃)Alkyl]₂, -C(O)OH, -C(O)O(C₁-C₃)Alkyl, -C(O)NH₂, -C(O)NH(C₁-C₃)Alkyl, - C(O)N[(C₁-C₃)Alkyl]₂, -SO₂NH₂, -SO₂NH(C₁-C₃)Alkyl, -SO₂N[(C₁-C₃)Alkyl]₂, - CN, (C₁-C₁₂)Alkyl und (C₅-C₈)Cycloalkyl, bedeutet,

6. Verfahren nach einem der Ansprüche 1 bis 5, **dadurch gekennzeichnet, dass** das Kation des Zyklisierungskatalysators ein Phosphoniumkation der allgemeinen Formel (XII) ist, in dem die Reste R¹⁶ bis R¹⁹ beziehungsweise R^{16'} bis R^{19'} (C₁ bis C₁₀)Alkyl bedeuten und insbesondere gleiche Alkylreste sind.

7. Verfahren nach Anspruch 6, **dadurch gekennzeichnet, dass** das Kation des Zyklisierungskatalysators der allgemeinen Formel (XII) gleich Tetra-(n)-butylphosphonium ist.

8. Verfahren nach einem der Ansprüche 1 bis 3, **dadurch gekennzeichnet, dass** das Anion des Zyklisierungskatalysators das Anion eines zyklisches Imids der allgemeinen Formel (VIII) oder (IX) ist.

9. Verfahren nach einem der Ansprüche 1 bis 3, **dadurch gekennzeichnet, dass** das Anion des Zyklisierungskatalysators das Anion eines Oxazolidinons der allgemeinen Formel (VIII) ist.

10. Verfahren nach Anspruch 2, **dadurch gekennzeichnet, dass** H-B in der allgemeinen Formel (IV) und das protonierte Anion des Katalysators der allgemeinen Formeln (VIII) bis (XI) den gleichen Heterocyclus darstellen.

11. Verfahren nach Anspruch 8, **dadurch gekennzeichnet, dass** der Heterocyclus ein Fünfringheterocyclus ist, der mindestens ein N im Ring und gegebenenfalls noch ein O im Ring enthält.

## Claims

1. A process for preparing 1,4-diphenylazetidinone derivatives from β-substituted amino amides which are protected in a suitable way in the presence of silylating agents and at least one cyclization catalyst, this cyclization catalyst being represented by one of the general formulae below 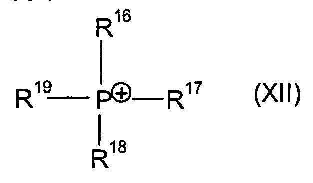 as cation, where R¹⁶, R¹⁷, R¹⁸, R¹⁹ are independently of one another aryl, (C₁-C₁₅)alkyl, benzyl,
R⁴¹ is aryl, (C₁-C₁₅)alkyl, benzyl,
R⁴² is (C₁-C₁₅)alkyl, benzyl, (C₅-C₈)cycloalkyl, aryl, where aryl may be substituted by F, Cl, Br, I, -OH, -O(C₁-C₃)alkyl, -NH₂, -NH(C₁-C₃)alkyl, -N[(C₁-C₃)alkyl]₂, -C(O)OH, -C(O)O(C₁-C₃)alkyl, -C(O)NH₂, -C(O)NH(C₁-C₃)alkyl, -C(O)N[(C₁-C₃)alkyl]₂, -SO₂NH₂, -SO₂NH(C₁-C₃)alkyl, -SO₂N[(C₁-C₃)alkyl]₂, -CN, (C₁-C₁₂)alkyl and (C₅-C₈)cycloalkyl,
and 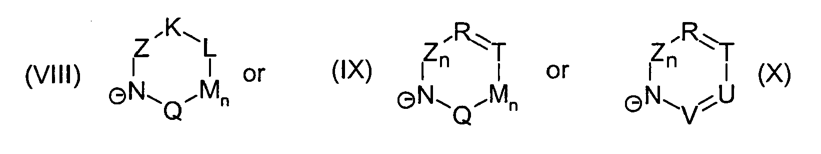 or 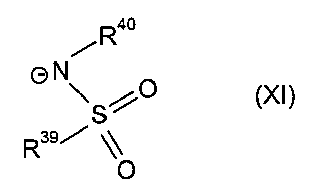 as anion,
and the symbols, substituents and indices have the following meaning,
Z = C=O, C=S, S=O, SO₂ or C=NR²⁰
K = O, S, NR²¹ or CR²²R²³
L = NR²⁴ or CR²⁵R²⁶
n = 0 or 1
M = O, C=O, NR²⁷ or CR²⁸R²⁹
Q = O, S, NR³⁰, CR³¹R³², C=O, C=S, S=O, SO₂ or C=NR³⁴
R = CR³⁵ or N
T = CR³⁶ or N
U = CR³⁷ or N
V = CR³⁸ or N
where R²⁰ to R³² and R³⁴ to R³⁸ are independently of one another H, (C₁-C₆)alkyl, aryl or heteroaryl, and in each case two alkyl radicals may together also form a cycloalkylene radical with a maximum of 6 C units in the ring which may in turn be substituted by F, Cl, Br, l, CF₃, NO₂, COO(C₁-C₆)alkyl, CON[(C₁-C₆)alkyl]₂, cycloalkyl, (C₁-C₁₀)alkyl, (C₂-C₆)alkenyl, O-(C₁-C₆)alkyl, O-CO-(C₁-C₆)alkyl, O-CO-(C₁-C₆)alkylene-aryl, SO₂N[(C₁-C₆)alkyl]₂, S-(C₁-C₆)alkyl, S-(CH₂-)ₙaryl, SO-(C₁-C₆)alkyl, SO-(CH₂-)ₙaryl, S0₂-(C₁-C₆)alkyl, SO₂-(CH₂-)ₙaryl, SO₂-N((C₁-C₆)alkyl)-(CH₂-)ₙaryl, or SO₂-N((CH₂-)ₙaryl)₂, where n may be 0 to 6, and the aryl radical may be substituted up to twice by F, Cl, Br, CF₃, SF₅, NO₂, OCF₃, O-(C₁-C₆)alkyl or (C₁-C₆)alkyl; or by N((C₁-C₆)alkyl)₂, NH-CO-NH-(C₁-C₆)alkyl), NH-CO-NH-aryl, N[(C₁-C₆)alkyl]-CO-(C₁-C₆)alkyl, N[(C₁-C₆)alkyl]-COO-(C₁-C₆)alkyl, N[(C₁-C₆)alkyl]-CO-aryl, N[(C₁-C₆)alkyl]-COO-aryl, N[(C₁-C₆)alkyl]-CON((C₁-C₆)alkyl)₂, N[(C₁-C₆)alkyl]-CO-N((C₁-C₆)alkyl)-aryl, N[(C₁-C₆)alkyl]-CO-N(aryl)₂, N(aryl)-CO-(C₁-C₆)alkyl, N(aryl)-COO-(C₁-C₆)alkyl, N(aryl)-CO-aryl, N(aryl)-COO-aryl, N(aryl)-CON((C₁-C₆)alkyl)₂, N(aryl)-CO-N[(C₁-C₆)alkyl]-aryl, N(aryl)-CON(aryl)₂, aryl, O-(CH2-)ₙaryl, where n may be 0 to 6, where the aryl radical may be substituted 1 to 3 times by F, Cl, Br, l, CF₃, NO₂, OCF₃, O-(C₁-C₆)alkyl, (C₁-C₆)alkyl, N((C₁-C₆)alkyl)₂, SF₅, SO₂-CH₃ or COO-(C₁-C₆)alkyl
and where R³⁹ and R⁴⁰ are independently of one another (C₁-C₆)alkyl, where one or more nonadjacent C atoms may be replaced by NH or C=O, or are (C₁-C₆)perfluoroalkyl, aryl or heteroaryl, or R³⁹ and R⁴⁰ form together a 1,8-naphthyl or 1,7,7-trimethylbicyclo[2.2.1]heptanyl, R⁴⁰ may also be H,
or where the cation in this cyclization catalyst corresponds to that of the general formula (XII), and the anion is R⁴¹O⁻' or where the cation in this cyclization catalyst corresponds to that of the general formula (XII) and the anion is R⁴²COO⁻, or where the cation in this cyclization catalyst corresponds to the general formula (XII), and the anion is Cl⁻, Br⁻ or I⁻, and these are combined with Ag₂O.

2. The process for preparing 1,4-diphenylazetidinone derivatives as claimed in claim 1, where these are represented by the general formula (1) 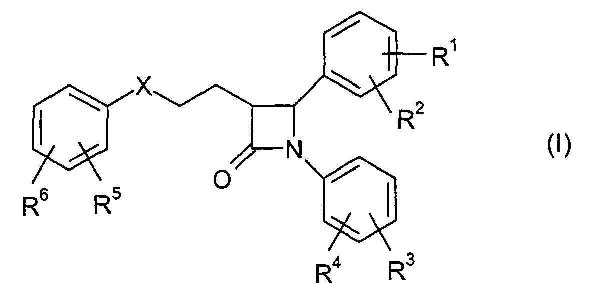 in which the symbols, substituents and indices have the following meaning,
X = CH₂, CHOH, CO or CHOCOR¹¹
R¹, R² = independently of one another H, OH, OCF₃, or O-(C₁-C₆)alkyl, O-(C₃-C₇)cycloalkyl, O-COR¹¹, CN, CH₂NHR⁷, CH₂NR⁷R⁸, NR⁷R⁸, COR¹⁴, F or Cl
R³, R⁴ = independently of one another H, F, Cl, OH, OCF₃, O-(C₁-C₆)alkyl, O-(C₃-C₇)cycloalkyl, O-COR¹¹, CN, CH₂NHR⁷, CH₂NR⁷R⁸, NR⁷R⁸, COR¹⁴ or (C₁-C₆)alkyl
R⁵, R⁶ = independently of one another H, F, Cl, (C₁-C₆)alkyl, CF₃ or OCF₃
R⁷ = H, C(=O)-Y(-CH₂)ₖ-Y-C(=O)R⁹ or C(=O)-Y(-CH₂)ₖ-NHR¹⁰
k = 2 to 16
Y = single bond or NR¹³
R⁸ = H, (C₁-C₆)alkyl, or (C₃-C₇)cycloalkyl
R⁹ = OH or NHCH₂[-CH(OH)]ₘ-CH₂OH or a form thereof protected in a suitable way
R¹⁰ = H, C(=O)[-CH(OH)]ₘ-CH₂OH or a form thereof protected in a suitable way
m = 0 to 5
R¹¹ = H, (C₁-C₆)alkyl, (C₃-C₇)cycloalkyl, (substituted) phenyl or OR¹²
R¹² = (C₁-C₆)alkyl or (C₃-C₇)cycloalkyl
R¹³ = (C₁-C₆)alkyl or (C₃-C₇)cycloalkyl, aryl or heteroaryl
R¹⁴ = OH, OR¹², NR¹³(-CH₂)ₙ-Y-C(=O)R⁹ or NR¹³(-CH₂)ₙ-NHR¹⁰
in the presence of a silylating agent and of a cyclization catalyst, wherein compounds of the general formula (IV) 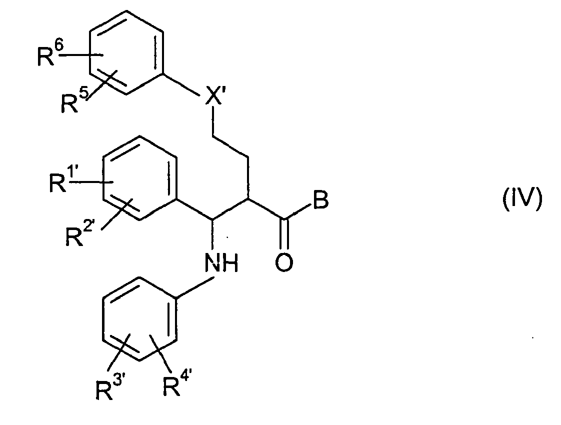 in which the symbols, substituents and indices - where not defined above - have the following meaning,
X' = X, CHOSi(alkyl)ₒ(aryl)ₚ with o, p = 1 to 3 and o + p = 3, C(Oalkyl)₂ or cyclic ketal such as C[O(-CH₂)_{q}-O] with q = 2, 3
R^{1'}, R^{2'} = R¹, R² and O-protective group
R^{3'}, R^{4'} = R³ R⁴, CH₂NHCO₂CH₂(C₆H₅), CH₂N[Si(alkyl)ₒ-(aryl)ₚ]CO₂CH₂(C₆H₅), CH₂NHCO₂-tert.Bu, CH₂N[Si(alkyl)ₒ(phenyl)p]CO₂-tert.Bu, CH₂NHC(C₆H₅)₃, CH₂N=C(C₆H₅)₂ or CH₂N=CH[C₆H₄(R⁶)] 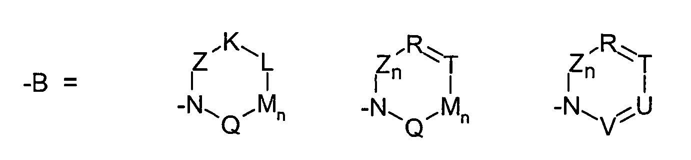
or 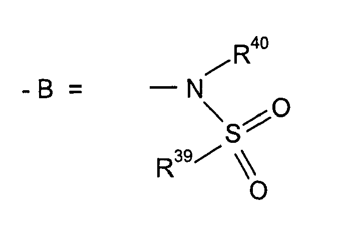 are cyclized to give (precursor) products of the general formula (V), which can be deprotected to give the compound (I) 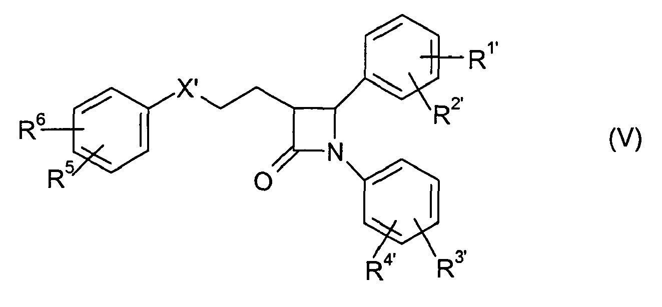 where the cyclization catalyst is represented by one of the general formulae below 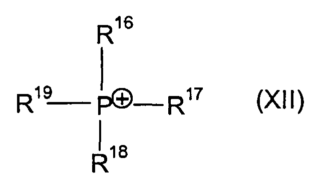 as cation, where R¹⁶, R¹⁷, R¹⁸, R¹⁹ are independently of one another aryl, (C₁-C₁₅)alkyl, benzyl,
R⁴¹ is aryl, (C₁-C₁₅)alkyl, benzyl,
R⁴² is (C₁-C₁₅)alkyl, benzyl, (C₅-C₈)cycloalkyl, aryl, where aryl may be substituted by F, CI, Br, I, -OH, -O(C₁-C₃)alkyl, -NH₂, -NH(C₁-C₃)alkyl, -N[(C₁-C₃)alkyl]₂, -C(O)OH, -C(O)O(C₁-C₃)alkyl, -C(O)NH₂, -C(O)NH(C₁-C₃)alkyl, -C(O)N[(C₁-C₃)alkyl]₂, -SO₂NH₂, -SO₂NH(C₁-C₃)alkyl, -SO₂N[(C₁-C₃)alkyl]₂, -CN, (C₁-C₁₂)alkyl and (C₅-C₈)cycloalkyl,
and 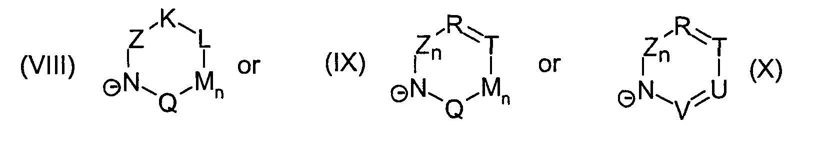 or 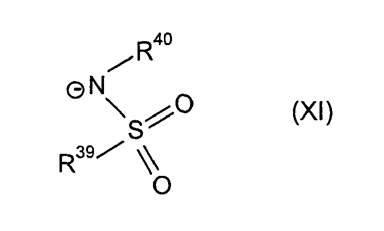 as anion,
or where the cation in this cyclization catalyst corresponds to that of the general formula (XII), and the anion is R⁴¹O⁻, or where the cation in this cyclization catalyst corresponds to that of the general formula (XII) and the anion is R⁴²COO⁻, or where the cation in this cyclization catalyst corresponds to the general formula (XII), and the anion is Cl⁻, Br⁻ or I , and these are combined with Ag₂O.

3. The process as claimed in claim 2, wherein compounds of the general formula (II) 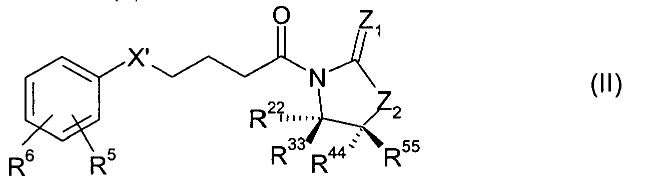 are reacted with imines of the general formula (III) 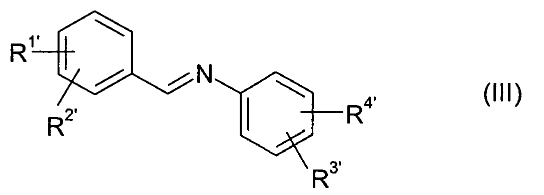 to give intermediates of the general formula (IV'), 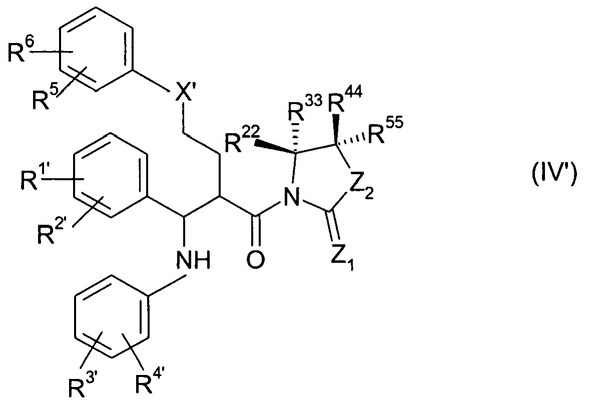 which are then cyclized to (precursor) products of the general formula (V), which can be deprotected to give the compound (I), 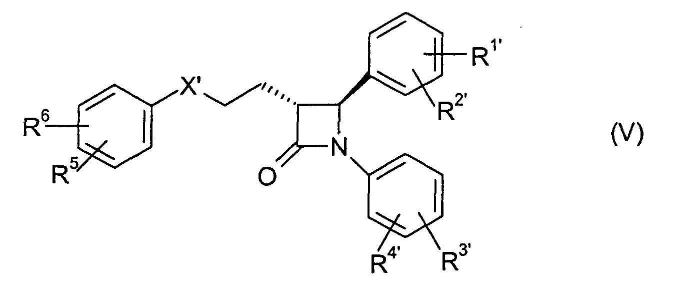 in which the symbols, substituents and indices - where not defined above - have the following meaning,
X' = X, CHOSi(alkyl)ₒ(aryl)ₚ with o, p = 1 to 3 and o + p = 3, C(Oalkyl)₂ or cyclic ketal such as C[O(-CH₂)_{q}-O] with q=2,3
R²², R³³, R⁴⁴, R⁵⁵ = independently of one another H, aryl or (C₁-C₁₀)alkyl
Z₁, Z₂ = independently of one another O, NH, NR¹⁵, or S
R¹⁵ = aryl or (C₁-C₁₀)alkyl,
R^{1'}, R^{2'}= R¹, R² and O-protective group
R^{3'}, R^{4'} = R³, R⁴, CH₂NHCO₂CH₂(C₆H₅), CH₂N[Si(alkyl)ₒ-(aryl)p]CO₂CH₂(C₆H₅), CH₂NHCO₂-tert.Bu, CH₂N[Si(alkyl)ₒ(phenyl)ₚ]CO₂-tert.Bu, CH₂NHC(C₆H₅)₃, CH₂N=C(C₆H₅)₂ or CH₂N=CH[C₆H₄(R⁶)]
where the cyclization catalyst is represented by one of the general formulae (VIa) to (VII) 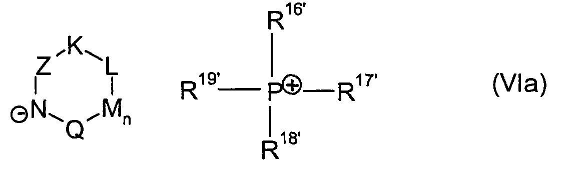 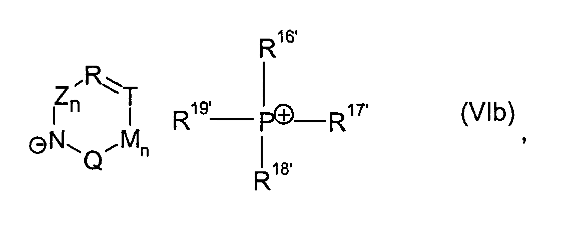 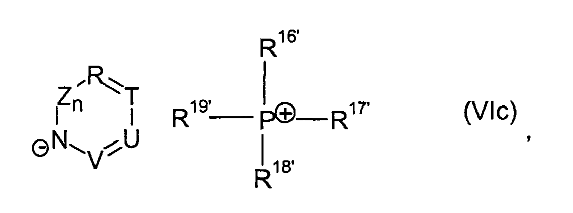 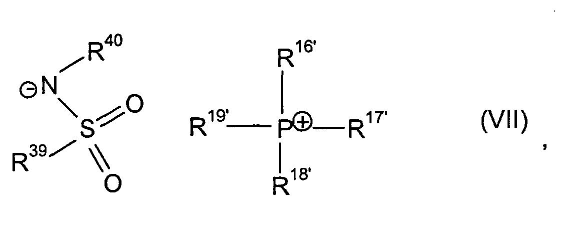 where, R^{16'}, R^{17'} , R^{18'}, R^{19'} are independently of one another aryl, (C₁-C₁₅)alkyl, benzyl.

4. The process as claimed in claim 2, wherein compounds of the general formula (II) 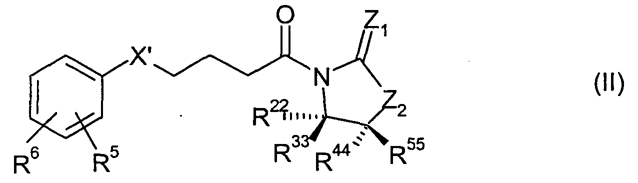 are reacted with imines of the general formula (III) 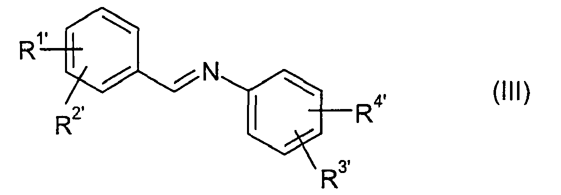 to give intermediates of the general formula (IV'), 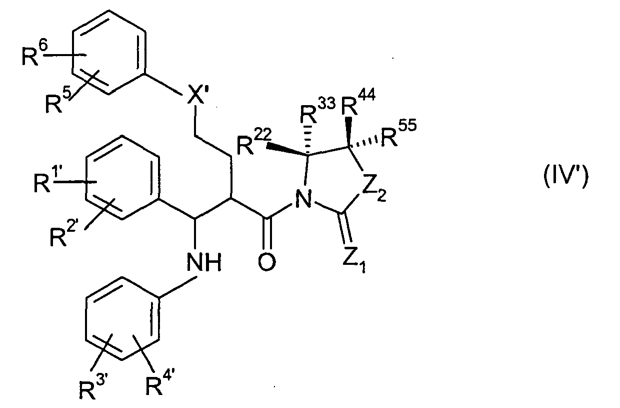 which are then cyclized to (precursor) products of the general formula (V), which can be deprotected to give the compound (I), 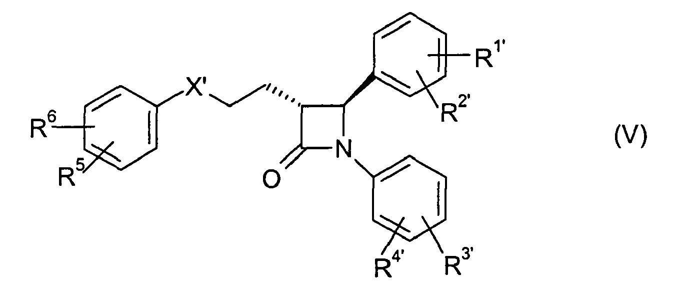 in which the symbols, substituents and indices - where not defined above - have the following meaning,
X' = X, CHOSi(alkyl)ₒ(aryl)ₚ with o, p = 1 to 3 and o + p = 3, C(Oalkyl)₂ or cyclic ketal such as C[O(-CH₂)_{q}-O] with q=2,3
R²², R³³, R⁴⁴, R⁵⁵ = independently of one another H, aryl or (C₁-C₁₀)alkyl
Z₁, Z₂ = independently of one another O, NH, NR¹⁵ or S
R¹⁵ = aryl or (C₁-C₁₀)alkyl,
R^{1'}, R^{2'}= R¹, R² and O-protective group
R^{3'},R^{4'} = R³, R⁴, CH₂NHCO₂CH₂(C₆H₅), CH₂N[Si(alkyl)ₒ-(aryl)ₚ]CO₂CH₂(C₆H₅), CH₂NHCO₂-tert.Bu, CH₂N[Si(alkyl)ₒ(phenyl)ₚ]CO₂-tert.Bu, CH₂NHC(C₆H₅)₃, CH₂N=C(C₆H₅)₂ or CH₂N=CH[C₆H₄(R⁶)]
where the cation in the cyclization catalyst corresponds to that of the general formula (XII), and the anion is R⁴¹O⁻, or where the cation in this cyclization catalyst corresponds to that of the general formula (XII), and the anion is R⁴²COO⁻, or where the cation in this cyclization catalyst corresponds to the general formula (XII), and the anion is Cl⁻, Br⁻ or I⁻, and these are combined with Ag₂O, as cation,
where R¹⁶, R¹⁷, R¹⁸, R¹⁹ are independently of one another aryl, (C₁-C₁₅)alkyl, benzyl,
R⁴¹ is aryl, (C₁-C₁₅)alkyl, benzyl,
R⁴² is (C₁-C₁₅)alkyl, benzyl, (C₅-C₈)cycloalkyl, aryl, where aryl may be substituted by F, Cl, Br, I, -OH, -O(C₁-C₃)alkyl, -NH₂, -NH(C₁-C₃)alkyl, -N[(C₁-C₃)alkyl]₂, -C(O)OH, -C(O)O(C₁-C₃)alkyl, -C(O)NH₂, -C(O)NH(C₁-C₃)alkyl, -C(O)N[(C₁-C₃)alkyl]₂, -SO₂NH₂, -SO₂NH(C₁-C₃)alkyl, -SO₂N[(C₁-C₃)alkyl]₂, -CN, (C₁-C₁₂)alkyl and (C₅-C₈)cycloalkyl,

5. The process as claimed in claim 2, wherein compounds of the general formula (II) 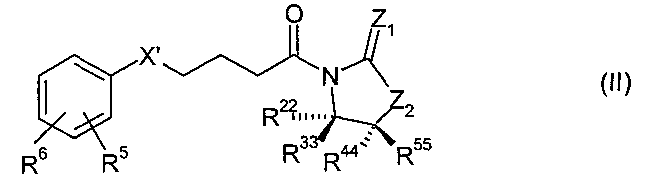 are reacted with imines of the general formula (III) 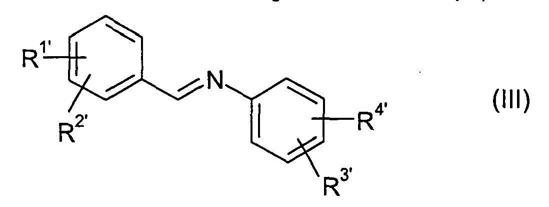 to give intermediates of the general formula (IV'), 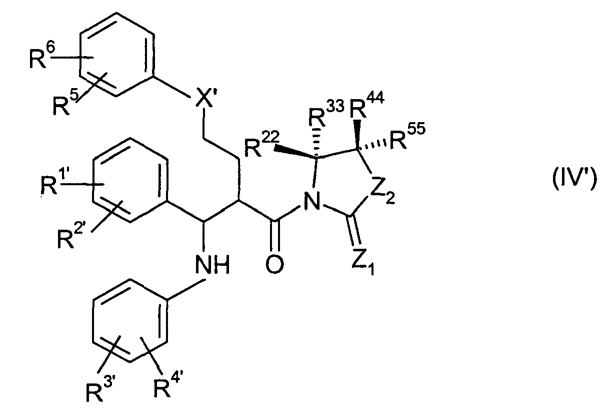 which are then cyclized to (precursor) products of the general formula (V), which can be deprotected to give the compound (I), 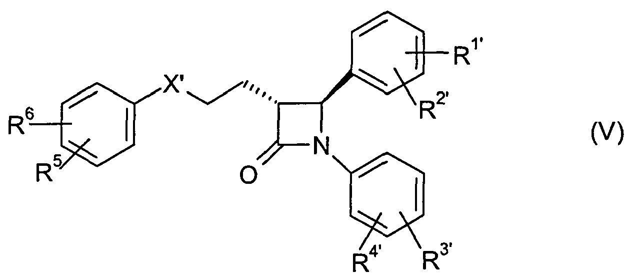 in which the symbols, substituents and indices - where not defined above - have the following meaning,
X' = X, CHOSi(alkyl)ₒ(aryl)ₚ with o, p = 1 to 3 and o + p = 3, C(Oalkyl)₂ or cyclic ketal such as C[O(-CH₂)_{q}-O] with q=2,3
R²², R³³, R⁴⁴, R⁵⁵ = independently of one another H, aryl or (C₁-C₁₀)alkyl
Z₁, Z₂ = independently of one another O, NH, NR¹⁵, or S
R¹⁵ = aryl or (C₁-C₁₀)alkyl,
R^{1'}, R^{2'}= R¹, R² and O-protective group
R^{3'}, R^{4'}= R³, R⁴, CH₂NHCO₂CH₂(C₆H₅), CH₂N[Si(alkyl)ₒ-(aryl)ₚ]CO₂CH₂(C₆H₅), CH₂NHCO₂-tert.Bu, CH₂N[Si(alkyl)ₒ(phenyl)ₚ]CO₂-tert.Bu, CH₂NHC(C₆H₅)₃, CH₂N=C(C₆H₅)₂ or CH₂N=CH[C₆H₄(R⁶)]
where the cation in the cyclization catalyst corresponds to that of the general formula (XII), and the anion is R⁴²COO⁻,
where R¹⁶, R¹⁷, R¹⁸, R¹⁹ are independently of one another aryl, (C₁-C₁₀)alkyl, benzyl;
R⁴¹ is aryl, (C₁-C₁₀)alkyl, benzyl,
R⁴² is (C₁-C₁₅)alkyl, benzyl, (C₅-C₈)cycloalkyl, aryl, where aryl may be substituted by F, Cl, Br, I, -OH, -O(C₁-C₃)alkyl, -NH₂, -NH(C₁-C₃)alkyl, -N[(C₁-C₃)alkyl]₂, -C(O)OH, -C(O)O(C₁-C₃)alkyl, -C(O)NH₂, -C(O)NH(C₁-C₃)alkyl, -C(O)N[(C₁-C₃)alkyl]₂, -SO₂NH₂, -SO₂NH(C₁-C₃)alkyl, -SO₂N[(C₁-C₃)alkyl]₂, -CN, (C₁-C₁₂)alkyl and (C₅-C₈)cycloalkyl,

6. The process as claimed in any of claims 1 to 5, wherein the cation of the cyclization catalyst is a phosphonium cation of the general formula (XII) in which the radicals R¹⁶ to R¹⁹ or R^{16'} to R^{19'} are (C₁ to C₁₀)alkyl and in particular are identical alkyl radicals.

7. The process as claimed in claim 6, wherein the cation of the cyclization catalyst of the general formula (XII) is equal to tetra-(n)-butylphosphonium.

8. The process as claimed in any of claims 1 to 3, wherein the anion of the cyclization catalyst is the anion of a cyclic imide of the general formula (VIII) or (IX).

9. The process as claimed in any of claims 1 to 3, wherein the anion of the cyclization catalyst is the anion of a oxazolidinone of the general formula (VIII).

10. The process as claimed in claim 2, wherein H-B in the general formula (IV) and the protonated anion of the catalyst of the general formulae (VIII) to (XI) represent the same heterocycle.

11. The process as claimed in claim 8, wherein the heterocycle is a five-membered heterocycle which comprises at least one N in the ring and where appropriate also an O in the ring.

## Revendications

1. Procédé de préparation de dérivés de 1,4-diphénylazétidinone à partir d'aminoamides β-substitués qui sont protégés de manière convenable en présence d'agents de silylation et d'au moins un catalyseur de cyclisation, ce catalyseur de cyclisation étant représenté par l'une des formules générales ci-dessous 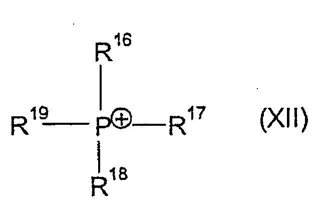 sous forme de cation, où R¹⁶, R¹⁷, R¹⁸, R¹⁹ sont indépendamment les uns des autres aryle, (C₁-C₁₅)alkyle, benzyle,
R⁴¹ est aryle, (C₁-C₁₅)alkyle, benzyle,
R⁴² est (C₁-C₁₅)alkyle, benzyle, (C₅-C₈)cycloalkyle, aryle, où aryle peut être substitué par F, Cl, Br, I, -OH, -O(C₁-C₃)alkyle, -NH₂, -NH(C₁-C₃)alkyle, -N[(C₁-C₃)alkyle]₂, -C(O)OH, -C(O)O(C₁-C₃)alkyle, -C(O)NH₂, -C(O)NH(C₁-C₃)alkyle, -C(O)N[(C₁-C₃)alkyle]₂, -SO₂NH₂, -S0₂NH(C₁-C₃)alkyle, -SO₂N[(C₁-C₃)alkyle]₂, -CN, (C₁-C₁₂)alkyle et (C₅-C₈)cycloalkyle,
et 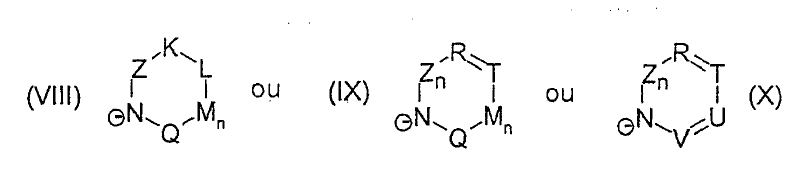 ou 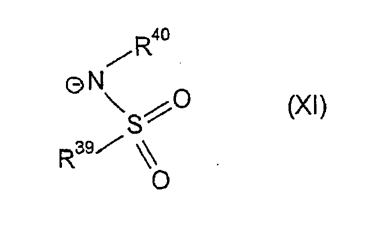 sous forme d'anion,
et les symboles, les substituants et les indices ont les significations suivantes,
Z = C=O, C=S, S=O, SO₂ ou C=NR²⁰
K = O, S, NR²¹ ou CR²²R²³
L = NR²⁴ ou CR²⁵R²⁶
n = 0 ou 1
M = O, C=O, NR²⁷ ou CR²⁸R²⁹
Q = O, S, NR³⁰, CR³¹R³², C=O, C=S, S=O, SO₂ ou C=NR³⁴
R = CR³⁵ ou N
T = CR³⁶ ou N
U = CR³⁷ ou N
V = CR³⁸ ou N
où R²⁰ à R³² et R³⁴ à R³⁸ sont indépendamment les uns des autres H, (C₁-C₆)alkyle, aryle ou hétéroaryle et dans chaque cas deux radicaux alkyle peuvent ensemble également former un radical cycloalkylène avec au maximum 6 unités de C dans le cycle qui peut à son tour être substitué par F, Cl, Br, l, CF₃, NO₂, COO(C₁-C₆)alkyle, CON[(C₁-C₆)alkyle]₂, cycloalkyle, (C₁-C₁₀)alkyle, (C₂-C₆)alcényle, O-(C₁-C₆)alkyle, O-CO-(C₁-C₆)alkyle, O-CO-(C₁-C₆)alkylène-aryle, SO₂N[(C₁-C₆)alkyle]₂, S-(C₁-C₆)alkyle, S-(CH₂-)ₙaryle, SO-(C₁-C₆)alkyle, SO-(CH₂-)ₙaryle, SO₂-(C₁-C₆)alkyle, SO₂-(CH₂-)ₙaryle, SO₂-N((C₁-C₆)alkyl)(CH₂-)ₙaryle, ou SO₂-N((CH₂-)ₙaryle)₂, où n peut être 0 à 6, et le radical aryle peut être substitué jusqu'à deux fois par F, Cl, Br, CF₃, SF₅, NO₂, OCF₃, O-(C₁-C₆)alkyle ou (C₁-C₆)alkyle ; ou par N((C₁-C₆)alkyle)₂, NH-CO-NH-(C₁-C₆)alkyle), NH-CO-NH-aryle, N[(C₁-C₆)alkyl]-CO-(C₁-C₆)alkyle, N[(C₁-C₆)alkyl]-COO-(C₁-C₆)alkyle, N[(C₁-C₆)alkyl]-CO-aryle, N[(Ci-C₆)alkyl]-COO-aryle, N[(C₁-C₆)alkyl]-CO-N((C₁-C₆)alkyle)₂, N[(C₁-C₆)alkyl]-CO-N((C₁-C₆)alkyl)-aryle, N[(C₁-C₆)alkyl]-CO-N(aryle)₂, N(aryl)-CO-(C₁-C₆)alkyle, N(aryl)-COO-(C₁-C₆)alkyle, N(aryl)-CO-aryle, N(aryl)-COO-aryle, N(aryl)-CO-N((C₁-C₆)alkyle)₂, N(aryl)-CON[(C₁-C₆)alkyl]-aryle, N(aryl)-CO-N(aryle)₂, aryle, O-(CH₂-)ₙaryle, où n peut être 0 à 6, où le radical aryle peut être substitué 1 à 3 fois par F, CI, Br, I, CF₃, NO₂, OCF₃, O-(C₁-C₆)alkyle, (C₁-C₆)alkyle, N((C₁-C₆)alkyle)₂, SF₅, SO₂-CH₃ ou COO-(C₁-C₆)alkyle
et où R³⁹ et R⁴⁰ sont indépendamment l'un de l'autre (C₁-C₆)alkyle, où un ou plusieurs atomes de C non adjacents peuvent être remplacés par NH ou C=O, ou sont (C₁-C₆)perfluoroalkyle, aryle ou hétéroaryle, ou R³⁹ et R⁴⁰ forment ensemble un 1,8-naphtyle ou 1,7,7-triméthylbicyclo[2,2,1]heptanyle, R⁴⁰ peut également être H,
ou où le cation dans ce catalyseur de cyclisation correspond à celui de formule générale (XII), et l'anion est R⁴¹O⁻ ou où le cation dans ce catalyseur de cyclisation correspond à celui de formule générale (XII), et l'anion est R⁴²COO⁻, ou où le cation dans ce catalyseur de cyclisation correspond à la formule générale (XII), et l'anion est Cl⁻, Br⁻ ou I⁻, et ceux-ci sont combinés avec de l'Ag₂O.

2. Procédé de préparation de dérivés de 1,4-diphénylazétidinone selon la revendication 1, **caractérisé en ce que** ceux-ci sont représentés par la formule générale (I) 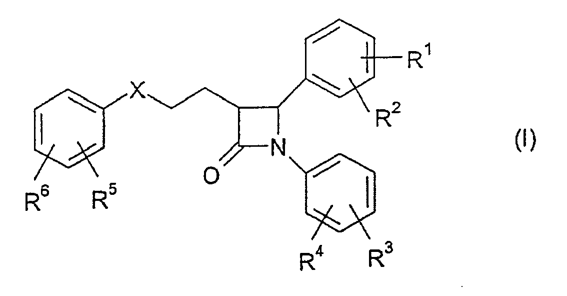 dans laquelle les symboles, les substituants et les indices ont les significations suivantes,
X = CH₂, CHOH, CO ou CHOCOR¹¹
R¹, R² = indépendamment l'un de l'autre H, OH, OCF₃, ou O-(C₁-C₆)alkyle, O-(C₃-C₇)cycloalkyle, O-COR¹¹, CN, CH₂NHR⁷, CH₂NR⁷R⁸, NR⁷R⁸, COR¹⁴, F ou CI
R³, R⁴ = indépendamment l'un de l'autre H, F, CI, OH, OCF₃, O-(C₁-C₆)alkyle, O-(C₃-C₇)cycloalkyle, O-COR¹¹, CN, CH₂NHR⁷, CH₂NR⁷R⁸, NR⁷R⁸, COR¹⁴ ou (C₁-C₆)alkyle
R⁵, R⁶ = indépendamment l'un de l'autre H, F, CI, (C₁-C₆)alkyle, CF₃ ou OCF₃
R⁷ = H, C(=O)-Y(-CH₂)ₖ-Y-C(=O)R⁹ ou C(=O)-Y(-CH₂)ₖ-NHR¹⁰
k = 2 à 16
Y = simple liaison ou NR¹³
R⁸ = H, (C₁-C₆)alkyle, ou (C₃-C₇)cycloalkyle
R⁹ = OH ou NHCH₂[-CH(OH)]ₘ-CH₂OH ou une forme de celui-ci protégée de manière convenable
R¹⁰ = H, C(=O)[-CH(OH)]ₘ-CH₂OH ou une forme de celui-ci protégée de manière convenable
m = 0 à 5
R¹¹ = H, (C₁-C₆)alkyle, (C₃-C₇)cycloalkyle, phényle (substitué) ou OR¹²
R¹² = (C₁-C₆)alkyle ou (C₃-C₇)cycloalkyle
R¹³ = (C₁-C₆)alkyle ou (C₃-C₇)cycloalkyle, aryle ou hétéroaryle
R¹⁴ = OH, OR¹², NR¹³(-CH₂)ₙ-Y-C(=O)R⁹ ou NR¹³(-CH₂)ₙ-NHR¹⁰
en présence d'un agent de silylation et d'un catalyseur de cyclisation, **caractérisé en ce que** les composés de formule générale (IV) 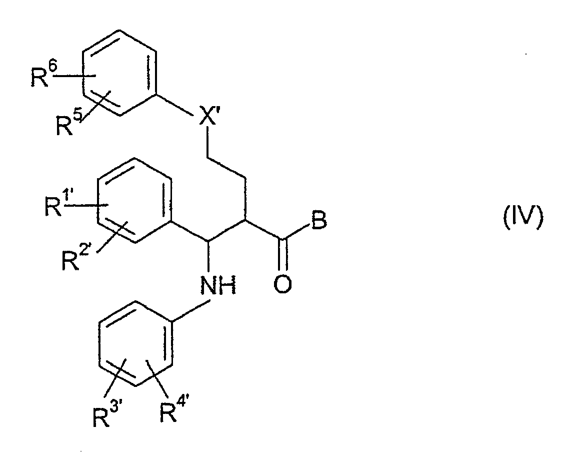 dans laquelle les symboles, les substituants et les indices - lorsqu'ils ne sont pas définis ci-dessus - ont la signification suivante,
X' = X, CHOSi(alkyl)ₒ(aryle)ₚ avec o, p = 1 à 3 et o + p = 3, C(Oalkyle)₂ ou cétal cyclique tel que C[O(-CH₂)_{q}-O] avec q = 2, 3
R^{1'}, R^{2'} = R¹, R² et groupement protecteur de O
R³', R^{4'} = R³, R⁴, CH₂NHCO₂CH₂(C₆H₅), CH₂N[Si(alkyl)ₒ(aryl)ₚ]-CO₂CH₂(C₆H₅), CH₂NHCO₂-tert.Bu, CH₂N[Si(alkyl)ₒ-(phényl)ₚ]CO₂-tert.Bu, CH₂NHC(C₆H₅)₃, CH₂N=C(C₆H₅)₂ ou CH₂N=CH[C₆H₄(R⁶)] 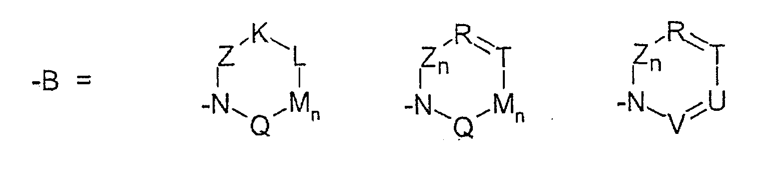
ou 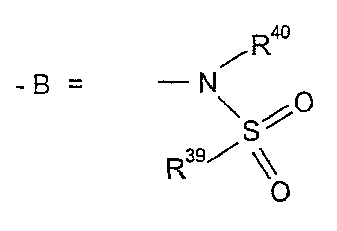 sont cyclisés pour donner des produits (précurseurs) de formule générale (V) qui peuvent être déprotégés pour donner le composé (I) 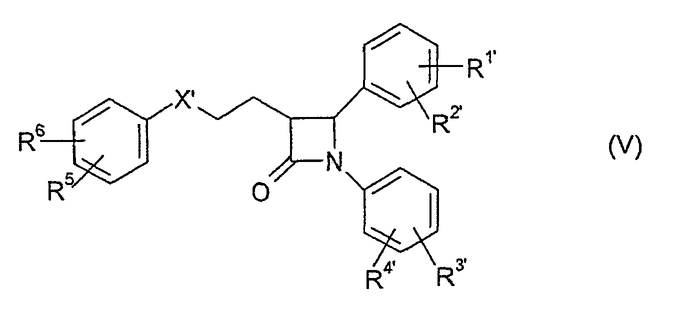 où le catalyseur de cyclisation est représenté par l'une des formules générales ci-dessous 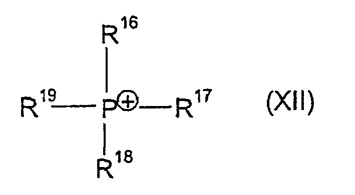 sous forme de cation, où R¹⁶, R¹⁷, R¹⁸, R¹⁹ sont indépendamment les uns des autres aryle, (C₁-C₁₅)alkyle, benzyle,
R⁴¹ est aryle, (C₁-C₁₅)alkyle, benzyle,
R⁴² est (C₁-C₁₅)alkyle, benzyle, (C₅-C₈)cycloalkyle, aryle, où aryle peut être substitué par F, Cl, Br, l, -OH, -O(C₁-C₃)alkyle, -NH₂, -NH(C₁-C₃)alkyle, -N[(C₁-C₃)alkyle]₂, -C(O)OH, -C(O)O(C₁-C₃)alkyle, -C(O)NH₂, -C(O)NH(C₁-C₃)alkyle, -C(O)N[(C₁-C₃)alkyle]₂, -SO₂NH₂, -SO₂NH(C₁-C₃)alkyle, -SO₂N[(C₁-C₃)alkyle]₂, -CN, (C₁-C₁₂)alkyle et (C₅-C₈)cycloalkyle,
et 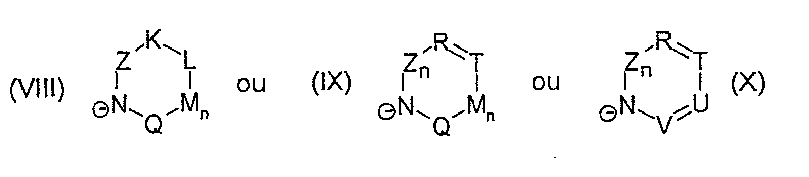 ou 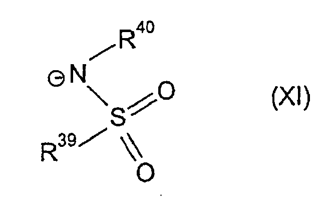 sous forme d'anion,
ou où le cation dans ce catalyseur de cyclisation correspond à celui de formule générale (XII), et l'anion est R⁴¹O⁻, ou où le cation dans ce catalyseur de cyclisation correspond à celui de formule générale (XII), et l'anion est R⁴²COO⁻ ou où le cation dans ce catalyseur de cyclisation correspond à la formule générale (XII), et l'anion est Cl⁻, Br⁻ ou l⁻, et ceux-ci sont combinés avec de l'Ag₂O.

3. Procédé selon la revendication 2, **caractérisé en ce que** des composés de formule générale (II) 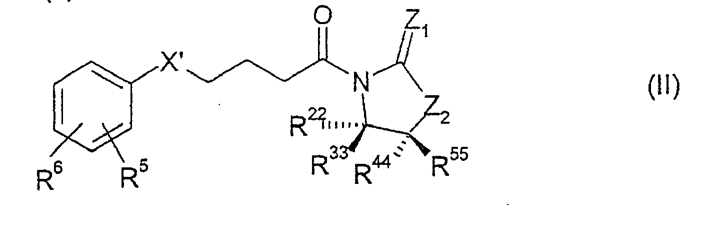 sont mis en réaction avec des imines de formule générale (III) 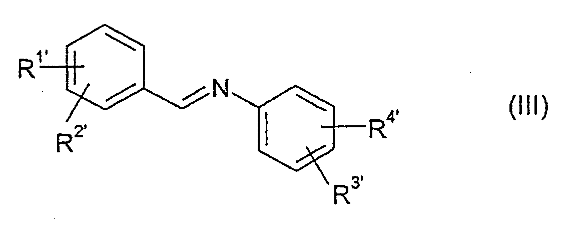 pour donner les intermédiaires de formule générale (IV'), 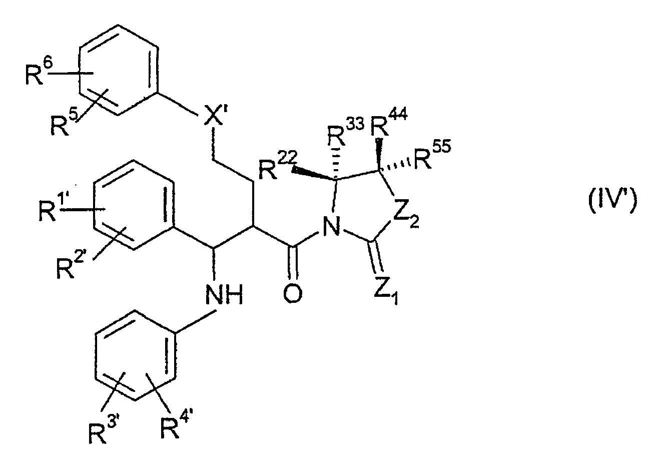 qui sont alors cyclisés pour donner les produits (précurseurs) de formule générale (V) qui peuvent être déprotégés pour donner le composé (1), 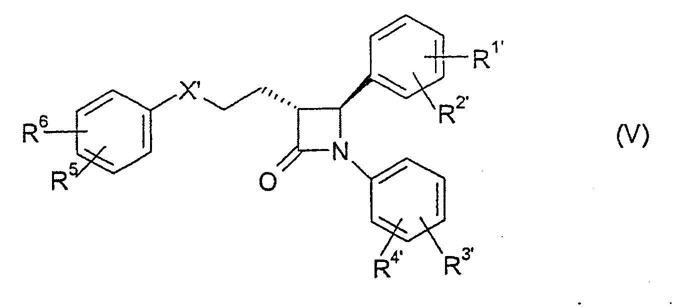 dans lequel les symboles, les substituants et les indices - lorsqu'ils ne sont pas définis ci-dessus - ont la signification suivante,
X' = X, CHOSi(alkyl)ₒ(aryle)ₚ avec o, p = 1 à 3 et o + p = 3, C(Oalkyle)₂ ou cétal cyclique tel que C[O(-CH₂)_{q}-O] avec q = 2, 3
R²², R³³, R⁴⁴, R⁵⁵ = indépendamment les uns des autres H, aryle ou (C₁-C₁₀)alkyle
Z₁, Z₂ = indépendamment l'un de l'autre O, NH, NR¹⁵, ou S
R¹⁵ = aryle ou (C₁-C₁₀)alkyle,
R^{1'}, R^{2'} = R¹, R² et groupement protecteur de O
R^{3'}, R^{4'} = R³, R⁴ CH₂NHCO₂CH₂(C₆H₅), CH₂N[Si(alkyl)ₒ(aryl)ₚ]-CO₂CH₂(C₆H₅), CH₂NHCO₂-tert.Bu, CH₂N[Si(alkyl)ₒ-(phényl)ₚ]CO₂-tert.Bu, CH₂NHC(C₆H₅)₃, CH₂N=C(C₆H₅)₂ ou CH₂N=CH[C₆H₄(R⁶)]
où le catalyseur de cyclisation est représenté par l'une des formules générales (VIa) à (VII) 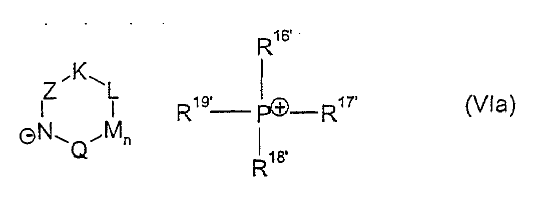 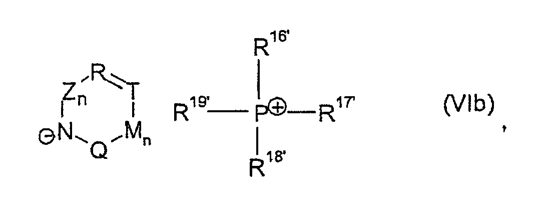 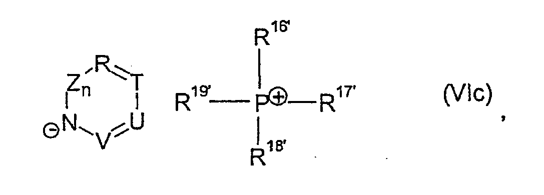 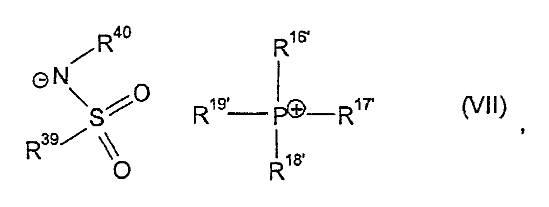 dans lesquelles R^{16'}, R^{17'}, R^{18'}, R^{19'} sont indépendamment les uns des autres aryle, (C₁-C₁₅)alkyle, benzyle.

4. Procédé selon la revendication 2, **caractérisé en ce que** des composés de formule générale (II) 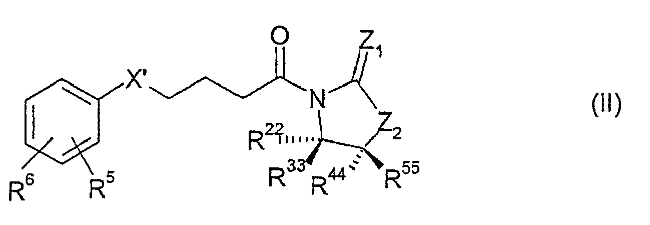 sont mis en réaction avec des imines de formule générale (III) 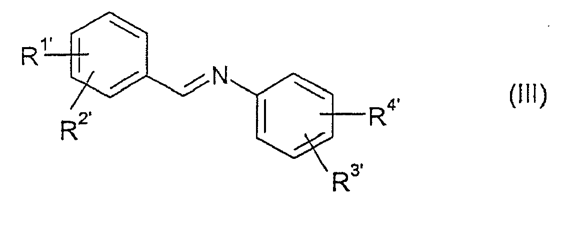 pour donner les intermédiaires de formule générale (IV'), 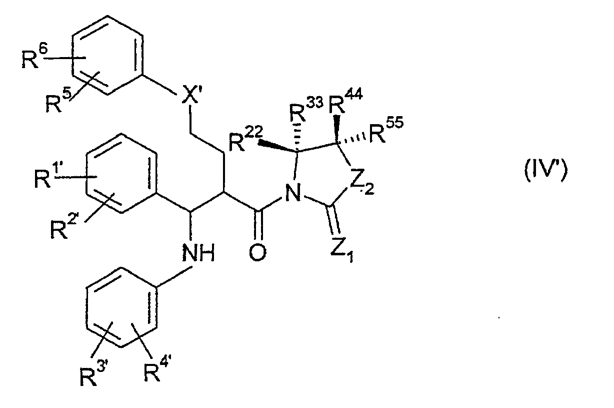 qui sont alors cyclisés pour donner les produits (précurseurs) de formule générale (V) qui peuvent être déprotégés pour donner le composé (I), 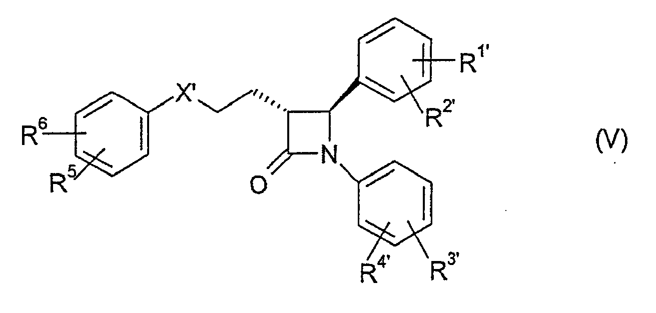 dans lequel les symboles, les substituants et les indices - lorsqu'ils ne sont pas définis ci-dessus - ont la signification suivante,
X' = X, CHOSi(alkyl)ₒ(aryle)ₚ avec o, p = 1 à 3 et o + p = 3, C(Oalkyle)₂ ou cétal cyclique tel que C[O(-CH₂)_{q}-O] avec q = 2, 3
R²², R³³, R⁴⁴, R⁵⁵ = indépendamment les uns des autres H, aryle ou (C₁-C₁₀)alkyle
Z₁, Z₂ = indépendamment l'un de l'autre O, NH, NR¹⁵, ou S
R¹⁵ = aryle ou (C₁-C₁₀)alkyle,
R^{1'}, R^{2'} = R¹, R² et groupement protecteur de O
R^{3'}, R^{4'} = R³, R⁴, CH₂NHCO₂CH₂(C₆H₅), CH₂N[Si(alkyl)ₒ(aryl)p]-CO₂CH₂(C₆H₅), CH₂NHCO₂-tert.Bu, CH₂N[Si(alkyl)ₒ-(phényl)ₚ]CO₂-tert.Bu, CH₂NHC(C₆H₅)₃, CH₂N=C(C₆H₅)₂ ou CH₂N=CH[C₆H₄(R⁶)]
où le cation dans le catalyseur de cyclisation correspond à celui de formule générale (XII), et l'anion est R⁴¹O⁻, ou où le cation dans ce catalyseur de cyclisation correspond à celui de formule générale (XII), et l'anion est R⁴²COO⁻ ou où le cation dans ce catalyseur de cyclisation correspond à la formule générale (XII), et l'anion est Cl⁻, Br⁻ ou I⁻, et ceux-ci sont combinés avec de l'Ag₂O, sous forme de cation,
où R¹⁶, R¹⁷, R¹⁸, R¹⁹ sont indépendamment les uns des autres aryle, (C₁-C₁₅)alkyle, benzyle,
R⁴¹ est aryle, (C₁-C₁₅)alkyle, benzyle,
R⁴² est (C₁-C₁₅)alkyle, benzyle, (C₅-C₈)cycloalkyle, aryle, où aryle peut être substitué par F, CI, Br, 1, -OH, -O(C₁-C₃)alkyle, -NH₂, -NH(C₁-C₃)alkyle, -N[(C₁-C₃)alkyle]₂, -C(O)OH, -C(O)O(C₁-C₃)alkyle, -C(O)NH₂, -C(O)NH(C₁-C₃)alkyle, -C(O)N[(C₁-C₃)alkyle]₂, -SO₂NH₂, SO₂NH(C₁-C₃)alkyle, -SO₂N[(C₁-C₃)alkyle]₂, -CN, (C₁-C₁₂)alkyle et (C₅-C₈)cycloalkyle.

5. Procédé selon la revendication 2, **caractérisé en ce que** des composés de formule générale (II) 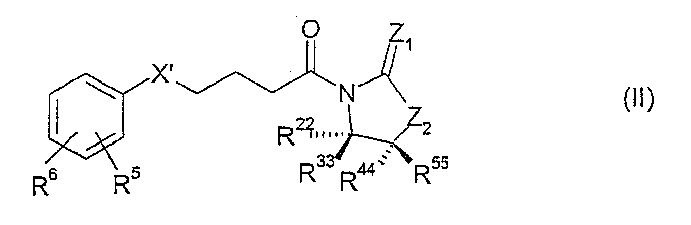 sont mis en réaction avec des imines de formule générale (III) 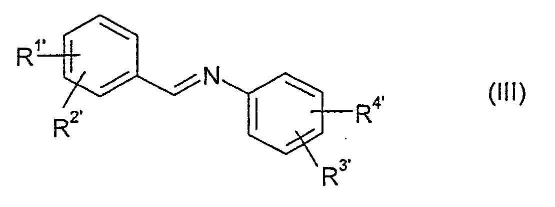 pour donner les intermédiaires de formule générale (IV'), 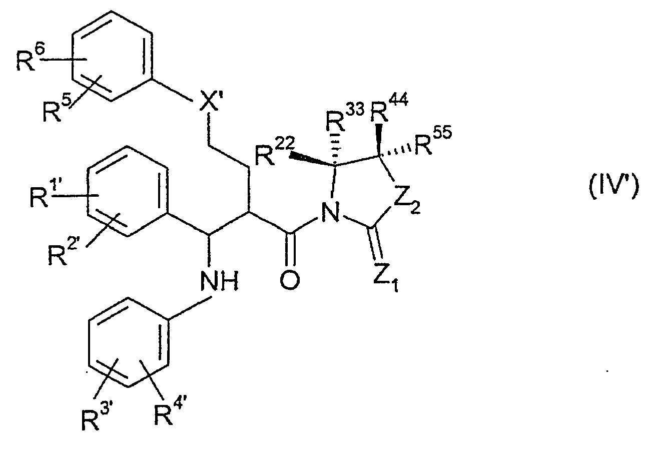 qui sont alors cyclisés pour donner les produits (précurseurs) de formule générale (V) qui peuvent être déprotégés pour donner le composé (I), 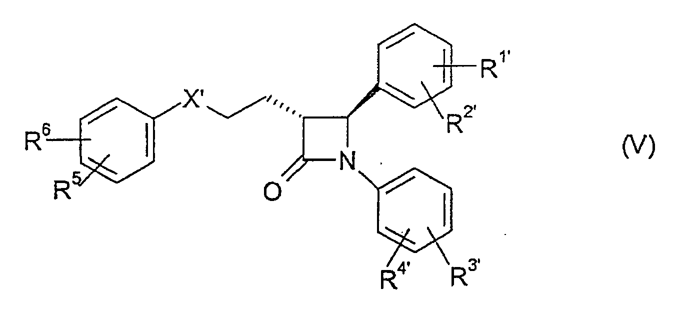 dans lequel les symboles, les substituants et les indices - lorsqu'ils ne sont pas définis ci-dessus - ont la signification suivante,
X' = X, CHOSi(alkyl)ₒ(aryle)ₚ avec o, p = 1 à 3 et o + p = 3, C(Oalkyle)₂ ou cétal cyclique tel que C[O(-CH₂)_{q}-O] avec q = 2, 3
R²², R³³, R⁴⁴, R⁵⁵ = indépendamment les uns des autres H, aryle ou (C₁-C₁₀)alkyle
Z₁, Z₂ = indépendamment l'un de l'autre O, NH, NR¹⁵, ou S
R¹⁵ = aryle ou (C₁-C₁₀)alkyle,
R^{1'}, R^{2'} = R¹, R² et groupement protecteur de O
R^{3'}, R^{4'} = R³, R⁴ CH₂NHCO₂CH₂(C₆H₅), CH₂N[Si(alkyl)ₒ(aryl)ₚ]-CO₂CH₂(C₆H₅), CH₂NHCO₂-tert.Bu, CH₂N[Si(alkyl)ₒ-(phényl)ₚ]CO₂-tert.Bu, CH₂NHC(C₆H₅)₃, CH₂N=C(C₆H₅)₂ ou CH₂N=CH[C₆H₄(R⁶)]
où le cation dans le catalyseur de cyclisation correspond à celui de formule générale (XII), et l'anion est R⁴²COO⁻,
où R¹⁶, R¹⁷, R¹⁸, R¹⁹ sont indépendamment les uns des autres aryle, (C₁-C₁₀)alkyle, benzyle ;
R⁴¹ est aryle, (C₁-C₁₀)alkyle, benzyle,
R⁴² est (C₁-C₁₅)alkyle, benzyle, (C₅-C₈)cycloalkyle, aryle, où aryle peut être substitué par F, Cl, Br, l, -OH, -O(C₁-C₃)alkyle, -NH₂, -NH(C₁-C₃)alkyle, -N[(C₁-C₃)alkyle]₂, -C(O)OH, -C(O)O(C₁-C₃)alkyle, -C(O)NH₂, -C(O)NH(C₁-C₃)alkyle, -C(O)N[(C₁-C₃)alkyle]₂, -SO₂NH₂, -SO₂NH(C₁-C₃)alkyle, -SO₂N[(C₁-C₃)alkyle]₂, -CN, (C₁-C₁₂)alkyle et (C₅-C₈)cycloalkyle.

6. Procédé selon l'une quelconque des revendications 1 à 5, **caractérisé en ce que** le cation du catalyseur de cyclisation est un cation phosphonium de formule générale (XII) dans laquelle les radicaux R¹⁶ à R¹⁹ ou R^{16'} à R^{19'} sont (C₁ à C₁₀)alkyle et en particulier sont des radicaux alkyle identiques.

7. Procédé selon la revendication 6, **caractérisé en ce que** le cation du catalyseur de cyclisation de formule générale (XII) est égal au tétra-(n)-butylphosphonium.

8. Procédé selon l'une quelconque des revendications 1 à 3, **caractérisé en ce que** l'anion du catalyseur de cyclisation est l'anion d'un imide cyclique de formule générale (VIII) ou (IX).

9. Procédé selon l'une quelconque des revendications 1 à 3, **caractérisé en ce que** l'anion du catalyseur de cyclisation est l'anion d'une oxazolidinone de formule générale (VIII).

10. Procédé selon la revendication 2, **caractérisé en ce que** H-B dans la formule générale (IV) et l'anion protoné du catalyseur de formules générales (VIII) à (XI) représentent le même hétérocycle.

11. Procédé selon la revendication 8, **caractérisé en ce que** l'hétérocycle est un hétérocycle à cinq chaînons qui comprend au moins un N dans le cycle et, le cas échéant, également un O dans le cycle.
